(19) Europäisches Patentamt
European Patent Office
Office européen des brevets

(11) EP 4 039 362 A1

(12) EUROPEAN PATENT APPLICATION
published in accordance with Art. 153(4) EPC

(43) Date of publication:
**10.08.2022 Bulletin 2022/32**

(21) Application number: **20871406.3**

(22) Date of filing: **10.09.2020**

(51) International Patent Classification (IPC):
***B01J 13/16*** *(2006.01)*

(52) Cooperative Patent Classification (CPC):
**B01J 13/16**

(86) International application number:
**PCT/JP2020/034221**

(87) International publication number:
**WO 2021/065400 (08.04.2021 Gazette 2021/14)**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**
Designated Validation States:
**KH MA MD TN**

(30) Priority: **30.09.2019 JP 2019178734**

(71) Applicant: **FUJIFILM Corporation**
**Tokyo 106-8620 (JP)**

(72) Inventors:
- **HAYASHI Shinya**
  **Fujinomiya-shi, Shizuoka 418-8666 (JP)**
- **ARIOKA Daisuke**
  **Fujinomiya-shi, Shizuoka 418-8666 (JP)**

(74) Representative: **Hoffmann Eitle**
**Patent- und Rechtsanwälte PartmbB**
**Arabellastraße 30**
**81925 München (DE)**

# (54) MICROCAPSULE, AND METHOD FOR MANUFACTURING MICROCAPSULE

(57) The present invention provides a microcapsule encompassing water or a water-soluble compound and having excellent leakage resistance and morphological stability. Further, the present invention provides a method for producing the microcapsule.

The microcapsule of the present invention is a microcapsule encompassing at least one of water or a water-soluble compound, in which a capsule wall of the microcapsule contains a resin having a repeating unit derived from a hydrophobic monomer having a ClogP value of 0.70 or more and a repeating unit derived from a hydrophilic monomer having a ClogP value of less than 0.70, and the microcapsule satisfies a relationship of Expression (1).

$$\text{Expression (1)}\ \delta/Dm \leq 0.40$$

$\delta$ represents a number average wall thickness ($\mu$m) of the microcapsule. Dm represents a volume-based median diameter ($\mu$m) of the microcapsule.

EP 4 039 362 A1

## Description

## BACKGROUND OF THE INVENTION

1. Field of the Invention

The present invention relates to a microcapsule and a method for producing a microcapsule.

2. Description of the Related Art

In recent years, a microcapsule has been attracting attention because the microcapsule can provide new value in terms of encompassing and protecting a functional material such as a fragrance, a dye, a heat storage material, a curing agent for an adhesive, or a pharmaceutical ingredient, and releasing the functional material in response to a stimulus.

For example, JP2015-164722A proposes "a microcapsule having a core-shell structure characterized in that a curing agent and/or a curing accelerator for an epoxy resin having a solubility in water of 5% by weight or more is coated with a polymer, ...."

JP2015-507529A proposes "a microcapsule dispersion including a microcapsule containing "a hydrophilic capsule core, ... (an omission of interior parts) ..., and a capsule wall polymer, in which the microcapsule is dispersed in a hydrophobic diluent".

## SUMMARY OF THE INVENTION

There is a requirement to encompass water or a water-soluble compound as a core of a microcapsule. With respect to existing microcapsules encompassing water or a water-soluble compound as a core (for example, the microcapsules described in JP2015-164722A and JP2015-507529A), the present inventors have found that there is room for improvement in the leakage resistance capable of suppressing leakage of the core ingredient or in the morphological stability of the microcapsule.

In view of the above circumstances, an object of the present invention is to provide a microcapsule encompassing water or a water-soluble compound and having excellent leakage resistance and morphological stability. Another object of the present invention is to provide a method for producing the microcapsule.

As a result of extensive studies on the foregoing objects, the present inventors have found that the foregoing objects can be achieved by the following configurations.

[1] A microcapsule encompassing at least one of water or a water-soluble compound, in which a capsule wall of the microcapsule contains a resin having a repeating unit derived from a hydrophobic monomer having a ClogP value of 0.70 or more and a repeating unit derived from a hydrophilic monomer having a ClogP value of less than 0.70, and

the microcapsule satisfies a relationship of Expression (1).

$$\text{Expression (1)} \qquad \delta/Dm \leq 0.40$$

$\delta$ represents a number average wall thickness ($\mu$m) of the microcapsule. Dm represents a volume-based median diameter ($\mu$m) of the microcapsule.

[2] The microcapsule according to [1], in which the median diameter is 7 to 200 $\mu$m.

[3] The microcapsule according to [1] or [2], in which a content of the repeating unit derived from the hydrophilic monomer is 32% by mass or less with respect to all the repeating units of the resin.

[4] The microcapsule according to any one of [1] to [3], in which the hydrophobic monomer includes a polyfunctional hydrophobic monomer.

[5] The microcapsule according to any one of [1] to [4], in which the hydrophilic monomer includes at least one monomer selected from the group consisting of a (meth)acrylic acid, a (meth)acrylic acid ester having a ClogP value of less than 0.70, and a (meth)acrylic acid amide having a ClogP value of less than 0.70.

[6] The microcapsule according to any one of [1] to [5], in which the hydrophobic monomer includes at least one monomer selected from the group consisting of a (meth)acrylic acid ester having a ClogP value of 0.70 or more and a styrene having a ClogP value of 0.70 or more.

[7] The microcapsule according to any one of [1] to [6], in which the hydrophobic monomer includes a (meth)acrylic

acid ester having a ClogP value of 1.90 or more.

[8] A method for producing the microcapsule according to any one of [1] to [7], the method including:

a step A1 of preparing a water phase containing the hydrophilic monomer and water;
a step B1 of preparing an oil phase containing the hydrophobic monomer and a water-insoluble solvent;
a step C of dispersing the water phase in the oil phase to prepare an emulsified liquid; and
a step D of polymerizing the hydrophobic monomer and the hydrophilic monomer to form the capsule wall to form the microcapsule encompassing the water.

[9] A method for producing the microcapsule according to any one of [1] to [7], the method including:

a step A2 of preparing a water phase containing water;
a step B2 of preparing an oil phase containing the hydrophobic monomer, the hydrophilic monomer, and a water-insoluble solvent;
a step C of dispersing the water phase in the oil phase to prepare an emulsified liquid; and
a step D of polymerizing the hydrophobic monomer and the hydrophilic monomer to form the capsule wall to form the microcapsule encompassing the water.

[10] The method for producing the microcapsule according to [8] or [9], in which the water phase further contains the water-soluble compound, and
the step D forms the microcapsule encompassing the water and the water-soluble compound.

[11] The method for producing the microcapsule according to any one of [8] to [10], in which a content of the hydrophilic monomer in the emulsified liquid is 1% to 25% by mass with respect to a total content of the hydrophobic monomer and the hydrophilic monomer.

According to an aspect of the present invention, it is possible to provide a microcapsule encompassing water or a water-soluble compound and having excellent leakage resistance and morphological stability. According to another aspect of the present invention, it is possible to provide a method for producing the microcapsule.

## BRIEF DESCRIPTION OF THE DRAWINGS

Fig. 1 is a micrograph of a cross section of a microcapsule in Example 12.
Fig. 2 is a micrograph of a cross section of a microcapsule in Comparative Example 2.

## DESCRIPTION OF THE PREFERRED EMBODIMENTS

Hereinafter, the present invention will be described in detail.

Any numerical range expressed by using "to" in the present specification means a range including the numerical values before and after the "to" as a lower limit value and an upper limit value, respectively.

In addition, in any numerical range described in a stepwise manner in the present specification, an upper limit value or a lower limit value described in a certain numerical range may be replaced with an upper limit value or a lower limit value of another numerical range described in a stepwise manner. In addition, in any numerical range described in the present specification, an upper limit value or a lower limit value described in a certain numerical range may be replaced with values shown in the Examples.

The various ingredients which will be described later may be used alone or in admixture of two or more thereof. For example, the hydrophilic monomer and/or the hydrophobic monomer which will be described later may be used alone or in admixture of two or more thereof.

In the present specification, the (meth)acrylate represents acrylate and methacrylate, and the (meth)acrylic represents acrylic and methacrylic.

In the present specification, the "preparation" means to include not only the act of preparing a specific material by synthesis and/or blending, but also the act of procuring a predetermined product by purchase or the like.

In the present specification, the "room temperature" means 25°C unless otherwise specified.

In the present specification, in a case of referring to a value that may vary with temperature, the value is a value at 25°C unless otherwise specified.

[Microcapsule]

The microcapsule according to the embodiment of the present invention is a microcapsule encompassing at least one of water or a water-soluble compound, in which a capsule wall of the microcapsule contains a resin having a repeating unit derived from a hydrophobic monomer having a ClogP value of 0.70 or more and a repeating unit derived from a hydrophilic monomer having a ClogP value of less than 0.70, and the microcapsule satisfies a relationship of Expression (1).

$$\text{Expression (1)}\ \delta/Dm \leq 0.40$$

$\delta$ represents a number average wall thickness ($\mu$m) of the microcapsule. Dm represents a volume-based median diameter ($\mu$m) of the microcapsule.

Although the mechanism by which the object of the present invention is achieved by using the microcapsule having the above-mentioned configuration is not always clear, the present inventors consider as follows.

That is, the microcapsule according to the embodiment of the present invention encompasses at least one of water or a water-soluble compound and has a capsule wall, the resin contained in the capsule wall (hereinafter, also referred to as "specific resin") has both a repeating unit derived from a hydrophilic monomer and a repeating unit derived from a hydrophobic monomer, and the microcapsule satisfies the expression of $\delta/Dm \leq 0.40$. It is considered that such a configuration of the microcapsule has made it possible to improve the leakage resistance of the inclusions while maintaining a high filling rate, since a core is water and/or a water-soluble compound, and a resin containing a repeating unit derived from a hydrophilic monomer having a high affinity with the core and a repeating unit derived from a hydrophobic monomer can protect the circumference of the core. Here, the filling rate means an amount (filling rate) of a target ingredient (water and/or water-soluble compound) encompassed by the microcapsule. In addition, it is considered that since the specific resin has an appropriate affinity with water and/or a water-soluble compound, as described above, which is not too strong, a decrease in the strength of the capsule wall due to excessive interaction of water and/or the water-soluble compound with the specific resin is unlikely to occur, leading to an improvement in morphological stability.

In addition, the microcapsule according to the embodiment of the present invention has good solvent resistance. It is considered that the reason is that the specific resin has both a repeating unit derived from a hydrophilic monomer and a repeating unit derived from a hydrophobic monomer, so that the affinity for an organic solvent is also adjusted to an appropriate range. In addition, it is considered that the dense structure of the capsule wall as described above also contributes to the excellent solvent resistance.

Hereinafter, it is also referred to as "the effect of the present invention is more excellent" in a case where any one or more of the leakage resistance, morphological stability, solvent resistance, and filling rate of the microcapsule are more excellent.

<Shape>

The volume-based median diameter (Dm) of the microcapsule according to the embodiment of the present invention is preferably 2 to 1,000 $\mu$m, more preferably 2 to 500 $\mu$m, and still more preferably 7 to 200 $\mu$m from the viewpoint that the effect of the present invention is more excellent. It is preferable that the volume-based median diameter (Dm) of the microcapsule is 2 $\mu$m or more from the viewpoint that the relationship of $\delta/D \leq 0.40$ can be easily satisfied.

The volume-based median diameter of the microcapsule can be controlled by adjusting the production conditions of the microcapsule or the like.

Here, the volume-based median diameter of the microcapsule refers to a diameter at which the total volume of particles on the large diameter side and the total volume of particles on the small diameter side are equal, in a case where the entire microcapsule is divided into two with the particle diameter at which the cumulative volume is 50% as the threshold value. That is, the median diameter corresponds to the so-called D50.

In the present specification, the volume-based median diameter of the microcapsule is measured using a laser diffraction/scattering-type particle size distribution analyzer LA-920 (manufactured by Horiba, Ltd.).

The number average wall thickness ($\delta$) of the microcapsule according to the embodiment of the present invention is preferably 0.05 to 200 $\mu$m, more preferably 0.1 to 100 $\mu$m, and still more preferably 0.2 to 50 $\mu$m.

The number average wall thickness of the microcapsule refers to a value obtained by measuring the thickness ($\mu$m) of each capsule wall of 20 microcapsules by a scanning electron microscope (SEM) and averaging the measured values.

Specifically, the number average wall thickness of the microcapsule is obtained by the following method. First, a liquid in which microcapsules are dispersed is applied onto a certain support which is then dried to form a coating film. A cross-sectional section of the obtained coating film is prepared, and the cross section thereof is observed at 100 to

5,000 times magnification by SEM to select any 20 microcapsules having a particle diameter in a range of "(value of volume-based median diameter of microcapsule) x 0.9 to (value of volume-based median diameter of microcapsule) x 1.1". Then, the cross section of each selected microcapsule is observed to obtain the thickness of the capsule wall and the average value of the obtained capsule wall thicknesses is calculated to obtain the number average wall thickness ($\delta$) of the microcapsule.

The microcapsule according to the embodiment of the present invention satisfies the relationship of Expression (1).

$$\text{Expression (1)} \qquad \delta/\text{Dm} \leq 0.40$$

$\delta$ represents the number average wall thickness ($\mu$m) of the microcapsule, the meaning of which is as described above. Dm represents the volume-based median diameter ($\mu$m) of the microcapsule, the meaning of which is as described above.

The value of $\delta$/Dm is 0.40 or less, preferably 0.01 to 0.40, more preferably 0.01 to 0.30, and particularly preferably 0.02 to 0.20.

In a case where the value of $\delta$/Dm is greater than 0.4, the wall is thick and the wall may contain a lot of inclusions, but in a case where the value of $\delta$/Dm is 0.4 or less, the inclusions contained in the wall are suppressed, which leads to an improvement in the leakage resistance of the inclusions. A smaller value of $\delta$/Dm, that is, a thinner wall thickness leads to a smaller amount of nuclei contained in the wall, which thus provides better leakage resistance.

The microcapsule according to the embodiment of the present invention is usually a mononuclear microcapsule.

The fact that the microcapsule is mononuclear means that there is substantially only one space in which the substance (core) encompassed by the microcapsule exists. The fact that there is substantially only one space in which the core exists means that one space (preferably a substantially spherical space) in the microcapsule, which has the largest size of the space in which the core exists, occupies 50% to 100% by volume (preferably 75% to 100% by volume and more preferably 95% to 100% by volume) with respect to the volume of the entire space in which the core exists. In addition, the volume of one space (preferably a substantially spherical space), which has the largest size of the space in which the core exists, is preferably 10 times or more larger than the volume of one space which has the second largest size.

In addition, the microcapsule according to the embodiment of the present invention is usually substantially spherical.

<Capsule wall>

The capsule wall in the microcapsule according to the embodiment of the present invention contains a resin (specific resin).

The content of the specific resin is preferably 50% to 100% by mass, more preferably 75% to 100% by mass, and still more preferably 95% to 100% by mass with respect to the total mass of the capsule wall.

The specific resin has a repeating unit derived from a hydrophobic monomer having a ClogP value of 0.70 or more (preferably 0.70 to 15.0) (hereinafter, also referred to as "hydrophobic unit") and a repeating unit derived from a hydrophilic monomer having a ClogP value of less than 0.70 (preferably -5.00 or more and less than 0.70) (hereinafter, also referred to as "hydrophilic unit").

The ClogP value is a value obtained by calculating a common logarithm logP of a 1-octanol-water partition coefficient P. The ClogP value is an indicator of hydrophilicity. Unless otherwise specified, the ClogP value in the present specification is a value calculated using the ClogP program incorporated in ChemBioDraw Professional 16.0 of Cambridge Soft Corporation.

The specific resin may contain both a hydrophobic unit and a hydrophilic unit, a ratio of the two units is not limited.

From the viewpoint that the effect of the present invention is more excellent, the content of the hydrophilic unit in the specific resin is preferably 70% by mass or less, more preferably 40% by mass or less, still more preferably 32% by mass or less, particularly preferably 25% by mass or less, and most preferably 10% by mass or less with respect to the total mass of the specific resin. The lower limit of the content of the hydrophilic unit is, for example, preferably 0.5% by mass or more, more preferably 1% by mass or more, and still more preferably 2% by mass or more.

Above all, in a case where the specific resin is a radical polymer which will be described later, the content of the hydrophilic unit in the specific resin is preferably 25% by mass or less and more preferably 10% by mass or less with respect to the total mass of the specific resin. The lower limit of the content of the hydrophilic unit is, for example, preferably 0.5% by mass or more, more preferably 1% by mass or more, and still more preferably 2% by mass or more.

The hydrophobic unit and the hydrophilic unit each may be one type or two or more types thereof may be used.

In addition, from the viewpoint that the effect of the present invention is more excellent, the content of the hydrophobic unit is preferably 30% by mass or more, more preferably 60% by mass or more, still more preferably 68%

by mass or more, particularly preferably 75% by mass or more, and most preferably 90% by mass or more with respect to the total mass of the specific resin. The upper limit of the content of the hydrophobic unit is, for example, preferably 99.5% by mass or less, more preferably 99% by mass or less, and still more preferably 98% by mass or less.

Above all, in a case where the specific resin is a radical polymer which will be described later, the content of the hydrophobic unit in the specific resin is preferably 75% by mass or more and more preferably 90% by mass or more with respect to the total mass of the specific resin. The upper limit of the content of the hydrophobic unit is, for example, preferably 99.5% by mass or less, more preferably 99% by mass or less, and still more preferably 98% by mass or less.

With regard to the content of each of the hydrophobic unit and the hydrophilic unit in the specific resin, the type of each repeating unit can be specified using a pyrolysis gas chromatograph mass spectrometer, and then a calibration curve can be created to calculate the content of each repeating unit.

In a case where the conditions for producing the microcapsule (such as the formulation amount of the hydrophilic monomer and the hydrophobic monomer) are known, the content of each of the hydrophobic unit and the hydrophilic unit may be obtained from the calculation.

The same applies to the content of the repeating unit derived from a polyfunctional monomer which will be described later.

The molecular weights of the hydrophobic monomer and the hydrophilic monomer are each independently preferably 25 to 2,000 and more preferably 40 to 1,500.

In addition, the molecular weight of each hydrophobic unit and the molecular weight of each hydrophilic unit are each independently preferably 25 to 2,000 and more preferably 40 to 1,500.

The total content of the hydrophobic unit and the hydrophilic unit satisfying the molecular weight range of 25 to 2,000 (preferably 40 to 1,500) is preferably 50% to 100% by mass, more preferably 75% to 100% by mass, and still more preferably 95% to 100% by mass with respect to the total mass of the specific resin.

From the viewpoint that the effect of the present invention is more excellent, it is also preferable to use a polyfunctional monomer selected from the group consisting of a polyfunctional hydrophobic monomer and a polyfunctional hydrophilic monomer.

Above all, it is more preferable that the hydrophobic monomer includes a polyfunctional hydrophobic monomer.

The monomer being polyfunctional is a monomer having 2 or 3 or more functional groups that serve as reaction points and enabling the introduction of a network structure into a polymer (resin) to be formed.

For example, the polyfunctional monomer has 2 or 3 or more (preferably 2 to 9) groups that are the groups shown in the functional group 1 or the functional group 2 which will be described later, and that can act as reaction points for the polymerization of the specific resin.

More specifically, for example, in a case where the specific resin is formed by radical polymerization, it can be said that, in a case where a monomer has 2 or more polymerizable double bonds as functional groups that serve as reaction points, the monomer is a polyfunctional monomer.

In addition, for example, in a case where a monomer has only functional groups which are sequentially polymerized in a one-to-one relationship with functional groups of another monomer as reaction points, it can be said that, in a case where the monomer has three or more such functional groups, the monomer is polyfunctional.

The specific resin preferably has a repeating unit derived from the polyfunctional monomer, and more preferably has a repeating unit derived from the polyfunctional hydrophobic monomer.

The content of the repeating unit derived from the polyfunctional monomer is preferably 5% to 100% by mass, more preferably 10% to 100% by mass, and still more preferably 20% to 100% by mass with respect to the total mass in the specific resin.

The polyfunctional monomer may be, for example, a polyfunctional radically polymerizable monomer which will be described later.

Above all, the content of the repeating unit derived from the polyfunctional hydrophobic monomer is preferably 5% to 100% by mass, more preferably 10% to 100% by mass, and still more preferably 20% to 100% by mass with respect to the total mass of the hydrophobic unit.

The polyfunctional hydrophobic monomer may be, for example, a polyfunctional hydrophobic radically polymerizable monomer which will be described later.

The specific resin is not limited as long as it is a resin obtained by polymerizing a hydrophobic monomer and a hydrophilic monomer. The specific resin may be a resin obtained by copolymerizing two or more monomers having a polymerizable double bond by chain polymerization such as a radical polymer ((meth)acrylic resin), or may be a resin obtained by step-growth polymerization such as polyamide and polyurea.

Examples of the resin that can be used for the specific resin include a radical polymer (such as a (meth)acrylic resin or a styrene-based resin), a resin having a urethane bond and/or a urea bond (such as polyurethane, polyurea, or polyurethane urea), a polyamide, a polyester, and a polyimide.

In addition, the specific resin may be, for example, a resin formed by reacting one or more combinations of the following (functional group 1/functional group 2) between different monomers.

(Functional group 1/functional group 2) = (polymerizable double bond/polymerizable double bond), (polymerizable double bond/thiol group), (carboxylic acid halide group (such as carboxylic acid chloride group)/primary or secondary amino group), (carboxyl group/primary or secondary amino group) (carboxylic acid anhydride group/primary or secondary amino group), (carboxyl group/aziridine group), (carboxyl group/isocyanate group), (carboxyl group/epoxy group), (carboxyl group/benzyl halide group), (primary or secondary amino group/isocyanate group), (primary, secondary, or tertiary amino group/benzyl halide group), (primary amino group/aldehydes), (isocyanate group/isocyanate group), (isocyanate group/hydroxyl group), (isocyanate group/epoxy group), (hydroxyl group/benzyl halide group), (hydroxyl group/carboxylic acid anhydride group), (hydroxyl group/alkoxysilyl group), (epoxy group/primary or secondary amino group), (epoxy group/carboxylic acid anhydride group), (epoxy group/hydroxyl group), (epoxy group/epoxy group), (oxetanyl group/epoxy group), (alkoxysilyl group/alkoxysilyl group), and the like.

The polymerizable double bond is intended to refer to a radically polymerizable carbon-to-carbon double bond, and examples thereof include carbon-to-carbon double bonds in a (meth)acryloyl group and a vinyl group.

In addition, in the above combination, the functional group 1 may be contained in the hydrophobic monomer and the functional group 2 may be contained in the hydrophilic monomer, or the functional group 2 may be contained in the hydrophobic monomer and the functional group 1 may be contained in the hydrophilic monomer. The hydrophobic monomer and/or the hydrophilic monomer may have both the functional group 1 and the functional group 2.

Above all, the specific resin is preferably one or more selected from the group consisting of a radical polymer, a resin having a urethane bond and/or a urea bond, a polyamide, and a polyester.

Hereinafter, these resins will be described in detail.

(Radical polymer)

The radical polymer is a resin formed by radical polymerization of a monomer containing a polymerizable double bond (ethylenic unsaturated group) capable of radical polymerization.

Examples of the monomer from which the radical polymer is derived (radically polymerizable monomer) include radically polymerizable monomers selected from the group consisting of (meth)acrylic acids, (meth)acrylic acid esters, (meth)acrylamides, (meth)acrylic acid halides, and styrenes.

The (meth)acrylic acid esters, (meth)acrylamides, (meth)acrylic acid halides, and styrenes each may be independently polyfunctional or non-polyfunctional (monofunctional).

The side chain of the specific resin, which is a radical polymer, may be modified.

Specific examples of the radically polymerizable monomer include the following monomers (hydrophobic radically polymerizable monomers or hydrophilic radically polymerizable monomers).

Examples of the radically polymerizable monomer having one polymerizable double bond include (meth)acrylic acids; (meth)acrylic acid esters such as 2-hydroxyethyl (meth)acrylate, 1-hydroxy-2-propyl (meth)acrylate, 2,3-dihydroxypropyl (meth)acrylate, hydroxymethyl (meth)acrylate, methyl (meth)acrylate, ethyl (meth)acrylate, propyl (meth)acrylate, butyl (meth)acrylate, and benzyl (meth)acrylate; (meth)acrylic acid amides such as acrylic acid amide, methacrylamide, and N,N-dimethylacrylic acid amide; acrylic acid halides such as (meth)acrylic acid chloride; and styrenes such as styrene, hydroxystyrene, and dihydroxystyrene.

Examples of the radically polymerizable monomer having two or more polymerizable double bonds include (poly)alkylene glycol di(meth)acrylates such as ethylene glycol di(meth)acrylate, 1,4-butanediol di(meth)acrylate, 1,6-hexanediol di(meth)acrylate, and 1,9-nonanediol di(meth)methacrylate; trimethylolpropane tri(meth)acrylate; pentaerythritol tri(meth)acrylate; dipentaerythritol penta(meth)acrylate; and dipentaerythritol hexa(meth)acrylate.

In a case where the specific resin is a radical polymer, the radically polymerizable monomer from which the radical polymer is derived has a hydrophobic unit derived from one or more of radically polymerizable monomers that satisfy the above-mentioned requirements as a hydrophobic monomer, and a hydrophilic unit derived from one or more of radically polymerizable monomers that satisfy the above-mentioned requirements as a hydrophilic monomer. Preferred conditions and the like as such a hydrophilic unit and a hydrophobic unit are as described above.

Above all, from the viewpoint that the effect of the present invention is more excellent, it is preferable that the hydrophobic monomer from which such a hydrophobic unit is derived includes at least one monomer (specific hydrophobic monomer) selected from the group consisting of (meth)acrylic acid esters having a ClogP value of 0.70 or more (preferably 0.70 to 15.00) and styrenes having a ClogP value of 0.70 or more (preferably 0.70 to 15.00).

Above all, it is more preferable that the hydrophobic monomer includes (meth)acrylic acid esters having a ClogP value of 1.90 or more (preferably 1.90 to 15.00, more preferably 2.30 to 12.00) as the specific hydrophobic monomer.

The content of the repeating unit derived from the specific hydrophobic monomer is preferably 50% to 100% by mass, more preferably 75% to 100% by mass, and still more preferably 95% to 100% by mass with respect to the total mass of the hydrophobic unit.

In addition, from the viewpoint that the effect of the present invention is more excellent, it is preferable that the hydrophilic monomer from which such a hydrophilic unit is derived includes at least one monomer (specific hydrophilic

monomer) selected from the group consisting of (meth)acrylic acid esters having a ClogP value of less than 0.70 (preferably -5.00 or more and less than 0.70) and (meth)acrylic acid amides having a ClogP value of less than 0.70 (preferably -5.00 or more and less than 0.70).

The content of the repeating unit derived from the specific hydrophilic monomer is preferably 50% to 100% by mass, more preferably 75% to 100% by mass, and still more preferably 95% to 100% by mass with respect to the total mass of the hydrophilic unit.

The radically polymerizable monomer preferably includes a polyfunctional radically polymerizable monomer (a radically polymerizable monomer having two or more polymerizable double bonds), and more preferably includes a polyfunctional and hydrophobic radically polymerizable monomer.

That is, it is also preferable that the specific resin, which is a radical polymer, has a repeating unit derived from a polyfunctional radically polymerizable monomer (preferably a polyfunctional and hydrophobic radically polymerizable monomer).

The polyfunctional radically polymerizable monomer is preferably a radically polymerizable monomer having 2 or more (preferably 2 to 9, more preferably 2 to 3) polymerizable double bonds.

In the specific resin which is a radical polymer, the content of the repeating unit derived from the polyfunctional radically polymerizable monomer is preferably 20% by mass or more, more preferably 30% by mass or more, still more preferably 30% to 98% by mass, and particularly preferably 30% to 80% by mass with respect to the total mass of the resin.

Above all, from the viewpoint that the effect of the present invention is more excellent, the specific resin is particularly preferably a resin containing 19% to 70% by mass of a repeating unit derived from monofunctional (meth)acrylic acid esters which are hydrophobic monomers having a ClogP value of 1.90 or more, 20% to 80% by mass of a repeating unit derived from polyfunctional (meth)acrylic acid esters which are hydrophobic monomers having a ClogP value of 1.90 or more, and 1% to 10% by mass of a repeating unit derived from (meth)acrylic acid esters which are hydrophilic monomers having a ClogP value of less than 0.70, with respect to the total mass of the resin.

(Resin having urethane bond and/or urea bond)

The specific resin may be a resin having a urethane bond and/or a urea bond.

Examples of the resin having a urethane bond and/or a urea bond include polyurethane, polyurea, and polyurethane urea.

The polyurethane is a polymer having a plurality of urethane bonds, and is preferably a reaction product formed from a raw material containing a polyol and a polyisocyanate.

In addition, the polyurea is a polymer having a plurality of urea bonds, and is preferably a reaction product formed from a raw material containing a polyamine and a polyisocyanate. It is also possible to synthesize the polyurea using a polyisocyanate without using a polyamine by utilizing the fact that a part of the polyisocyanate reacts with water to form the polyamine.

In addition, the polyurethane urea is a polymer having a plurality of urethane bonds and urea bonds, and is preferably a reaction product formed from a raw material containing a polyol, a polyamine, and a polyisocyanate. In a case where the polyol and the polyisocyanate are reacted, a part of the polyisocyanate reacts with water to form a polyamine, and as a result, polyurethane urea may be obtained.

The specific resin that is a resin having a urethane bond and/or a urea bond is preferably such that more than 50% by mass (preferably 75% to 100% by mass, more preferably 90% to 100% by mass) of the total mass of the resin is a repeating unit derived from a polyol, a polyamine, and a polyisocyanate bonded by a urethane bond and/or a urea bond.

The polyisocyanate is a compound having two or more isocyanate groups, and examples thereof include an aromatic polyisocyanate and an aliphatic polyisocyanates. The aromatic polyisocyanate is preferable from the viewpoint that an aromatic ring group can be introduced into a capsule wall of a microcapsule.

Examples of the aromatic polyisocyanate include aromatic diisocyanates, such as phenylene diisocyanate (for example, m-phenylene diisocyanate or p-phenylene diisocyanate), toluene diisocyanate (for example, 2,6-toluene diisocyanate or 2,4-toluene diisocyanate), naphthalene-1,4-diisocyanate, diphenylmethane-4,4'-diisocyanate, 3,3'-dimethoxy-biphenyldiisocyanate, 3,3'-dimethyldiphenylmethane-4,4'-diisocyanate, xylylene-1,4-diisocyanate, xylylene-1,3-diisocyanate, 4-chloroxylylene-1,3-diisocyanate, 2-methylxylylene-1,3-diisocyanate, 4,4'-diphenylpropane diisocyanate, and 4,4'-diphenylhexafluoropropane diisocyanate.

Examples of the aliphatic polyisocyanate include aliphatic diisocyanates, such as trimethylene diisocyanate, hexamethylene diisocyanate, propylene-1,2-diisocyanate, butylene-1,2-diisocyanate, cyclohexylene-1,2-diisocyanate, cyclohexylene-1,3-diisocyanate, cyclohexylene-1,4-diisocyanate, dicyclohexylmethane-4,4'-diisocyanate, 1,4-bis(isocyanatemethyl)cyclohexane, 1,3-bis(isocyanatemethyl)cyclohexane, isophorone diisocyanate, lysine diisocyanate, and xylylene diisocyanate hydride.

Although difunctional aromatic polyisocyanates and aliphatic polyisocyanates have been exemplified above, trifunctional or higher functional polyisocyanates (for example, trifunctional triisocyanates and tetrafunctional tetraisocyanates) can also be mentioned as the polyisocyanate.

More specifically, a biuret or isocyanurate which is a trimer of the above-mentioned difunctional polyisocyanate, an adduct of a polyol such as trimethylolpropane with a difunctional polyisocyanate, a formalin condensate of benzene isocyanate, polyisocyanate having a polymerizable group such as methacryloyloxyethyl isocyanate, and lysine triisocyanate can also be mentioned as the polyisocyanate.

Polyisocyanates are described in the "Polyurethane Resin Handbook" (edited by Keiji Iwata, published by The Nikkan Kogyo Shimbun, Ltd. (1987)).

Above all, a trifunctional or higher functional polyisocyanate (polyfunctional polyisocyanate) is preferable as one of the suitable aspects of the polyisocyanate.

Examples of the trifunctional or higher functional polyisocyanate include a trifunctional or higher functional aromatic polyisocyanate and a trifunctional or higher functional aliphatic polyisocyanate.

The trifunctional or higher functional polyisocyanate is preferably a trifunctional or higher functional polyisocyanate (an adduct type trifunctional or higher functional polyisocyanate) that is an adduct of an aromatic or alicyclic diisocyanate with a compound having three or more active hydrogen groups in one molecule (for example, a trifunctional or higher functional polyol, polyamine, or polythiol), or a trimer (biuret type or isocyanurate type) of an aromatic or alicyclic diisocyanate, and more preferably a trifunctional or higher functional polyisocyanate that is the adduct.

The trifunctional or higher functional polyisocyanate that is the adduct is preferably a trifunctional or higher functional polyisocyanate that is an adduct of an aromatic or alicyclic diisocyanate with a polyol having three or more hydroxyl groups in one molecule, and more preferably a trifunctional polyisocyanate that is an adduct of an aromatic or alicyclic diisocyanate with a polyol having three hydroxyl groups in one molecule.

As the adduct, it is preferable to use an adduct obtained by using an aromatic diisocyanate, from the viewpoint that the effect of the present invention is more excellent.

For example, the polyol is preferably a trifunctional or higher functional low molecular weight polyol which will be described later, and more preferably trimethylolpropane.

In addition, polymethylene polyphenyl polyisocyanate is also preferable as the polyisocyanate.

The polymethylene polyphenyl polyisocyanate is preferably a compound represented by Formula (X).

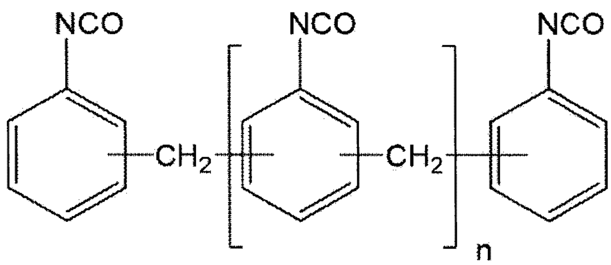

(X)

In Formula (X), n represents the number of repeating units. The number of repeating units represents an integer of 1 or more, and n is preferably an integer of 1 to 10 and more preferably an integer of 1 to 5.

The polyol is a compound having two or more hydroxyl groups, and examples thereof include a low molecular weight polyol (for example, an aliphatic polyol or an aromatic polyol), a polyvinyl alcohol, a polyether-based polyol, a polyester-based polyol, a polylactone-based polyol, a castor oil-based polyol, a polyolefin-based polyol, and a hydroxyl group-containing amine-based compound.

The low molecular weight polyol means a polyol having a molecular weight of 400 or less, and examples thereof include difunctional low molecular weight polyols such as ethylene glycol, diethylene glycol, and propylene glycol, and trifunctional or higher functional low molecular weight polyols such as glycerin, trimethylolpropane, hexanetriol, pentaerythritol, and sorbitol.

Examples of the hydroxyl group-containing amine-based compound include amino alcohols as oxyalkylated derivatives or the like of amino compounds. Examples of the amino alcohol include N,N,N',N'-tetrakis[2-hydroxypropyl]ethylenediamine and N,N,N',N'-tetrakis[2-hydroxyethyl]ethylenediamine, which are propylene oxide and ethylene oxide adducts of an amino compound such as ethylenediamine.

The polyamine is a compound having two or more amino groups (primary amino groups or secondary amino groups), and examples thereof include aliphatic polyvalent amines such as diethylenetriamine, triethylenetetramine, 1,3-propylenediamine, and hexamethylenediamine; epoxy compound adducts of aliphatic polyvalent amines; alicyclic polyvalent amines such as piperazine; and heterocyclic diamines such as 3,9-bis-aminopropyl-2,4,8,10-tetraoxaspiro-(5,5)undecane.

The polyisocyanate may be a hydrophobic monomer or a hydrophilic monomer, and is preferably a hydrophobic monomer. That is, the repeating unit derived from the polyisocyanate may be a hydrophobic unit or a hydrophilic unit, and is preferably a hydrophobic unit.

The polyol and the polyamine each may be independently a hydrophobic monomer or a hydrophilic monomer, and are each independently preferably a hydrophilic monomer. That is, the repeating unit derived from the polyol and the repeating unit derived from the polyamine each may be independently a hydrophobic unit or a hydrophilic unit, and are each independently preferably a hydrophilic unit.

Preferred conditions for such hydrophobic monomer and hydrophilic monomer, and hydrophobic unit and hydrophilic unit are as described above.

(Polyamide)

The specific resin may be a polyamide.

Examples of the polyamide include an aliphatic polyamide and an aromatic polyamide.

The polyamide is preferably, for example, a resin obtained by polymerizing a polyamine (diamine or the like) with a polycarboxylic acid (dicarboxylic acid or the like) and/or a polycarboxylic acid halide (dicarboxylic acid halide or the like).

Examples of the polycarboxylic acid halide include polycarboxylic acid chlorides (dicarboxylic acid chloride and the like).

The polycarboxylic acid may be a carboxylic acid anhydride in which carboxylic acid groups are dehydrated and condensed.

The specific resin that is a polyamide is preferably such that more than 50% by mass (preferably 75% to 100% by mass, more preferably 90% to 100% by mass) of the total mass of the resin is a repeating unit derived from a polyamine, a polycarboxylic acid, and a polycarboxylic acid halide bonded by an amide bond.

The polyamine from which the repeating unit of the specific resin that is a polyamide is derived is preferably a diamine.

Examples of the polyamine include an aliphatic polyamine, an alicyclic polyamine, and an aromatic polyamine.

Examples of the diamine include an aliphatic diamine, an alicyclic diamine, and an aromatic diamine.

For example, a diamine represented by General Formula (A) can be used as the aliphatic diamine or the alicyclic diamine. Ri in the following formula represents an aliphatic or alicyclic alkylene group represented by $C_nH_{2n}$ (n = 1 to 12).

$$H_2N\text{-}R_1\text{-}NH_2 \qquad (A)$$

Examples of the aromatic diamine include xylylenediamine and m-xylylenediamine.

The polycarboxylic acid from which the repeating unit of the specific resin that is a polyamide is derived is preferably a dicarboxylic acid.

Examples of the dicarboxylic acid include an aliphatic dicarboxylic acid, an alicyclic dicarboxylic acid, and an aromatic dicarboxylic acid.

For example, a dicarboxylic acid represented by General Formula (B) can be used as the aliphatic dicarboxylic acid or the alicyclic dicarboxylic acid. $R_2$ in the following formula represents an aliphatic or alicyclic alkylene group represented by $C_nH_{2n}$ (n = 4 to 25).

$$HOOC\text{-}R_2\text{-}COOH \qquad (B)$$

Examples of the aliphatic dicarboxylic acid include adipic acid.

Examples of the aromatic dicarboxylic acid include terephthalic acid, methyl terephthalic acid, naphthalenedicarboxylic acid, and isophthalic acid.

The aromatic dicarboxylic acid is preferably, for example, terephthalic acid or isophthalic acid from the viewpoint that it can exhibit excellent properties even at high temperatures.

The polycarboxylic acid halide (polycarboxylic acid chloride or the like) from which the repeating unit of the specific resin that is a polyamide is derived is preferably a dicarboxylic acid halide (dicarboxylic acid chloride or the like).

Examples of the dicarboxylic acid halide include compounds in which the carboxyl group (-COOH) in the above-mentioned dicarboxylic acid is replaced with a carboxylic acid halide group (-COX where X represents a halogen atom which is preferably a chlorine atom).

The polycarboxylic acid and the polycarboxylic acid halide each may be independently a hydrophobic monomer or a hydrophilic monomer, and are each independently preferably a hydrophobic monomer. That is, the repeating unit derived from the polycarboxylic acid and the repeating unit derived from the polycarboxylic acid halide each may be independently a hydrophobic unit or a hydrophilic unit, and are each independently preferably a hydrophobic unit.

The polyamines each may be independently a hydrophobic monomer or a hydrophilic monomer, and is preferably a hydrophilic monomer. That is, the repeating unit derived from the polyamine may be a hydrophobic unit or a hydrophilic unit, and is preferably a hydrophilic unit.
Preferred conditions for such hydrophobic monomer and hydrophilic monomer, and hydrophobic unit and hydrophilic unit are as described above.

(Polyester)

The specific resin may be a polyester.
Examples of the polyester include an aliphatic polyester and an aromatic polyester.
The polyester is preferably, for example, a resin obtained by polymerizing a polyol (diol or the like) with a polycarboxylic acid (dicarboxylic acid or the like) and/or a polycarboxylic acid halide (dicarboxylic acid halide or the like).
The specific resin that is a polyester is preferably such that more than 50% by mass (preferably 75% to 100% by mass, more preferably 90% to 100% by mass) of the total mass of the resin is a repeating unit derived from a polyol, a polycarboxylic acid, and a polycarboxylic acid halide bonded by an ester bond.
Examples of the polyol from which the repeating unit of the specific resin that is a polyester is derived include a compound in which the amine in the polyamine mentioned in the description of polyamide is replaced with a hydroxyl group.
In addition, the polyols mentioned in the description of the resin having a urethane bond and/or a urea bond may be used.
Examples of the polycarboxylic acid and polycarboxylic acid halide (polycarboxylic acid chloride or the like) from which the repeating unit of the specific resin that is a polyester is derived include the polycarboxylic acid and the polycarboxylic acid halide (polycarboxylic acid chloride or the like) mentioned in the description of polyamide.
The polycarboxylic acid and the polycarboxylic acid halide each may be independently a hydrophobic monomer or a hydrophilic monomer, and are each independently preferably a hydrophobic monomer. That is, the repeating unit derived from the polycarboxylic acid and the repeating unit derived from the polycarboxylic acid halide each may be independently a hydrophobic unit or a hydrophilic unit, and are each independently preferably a hydrophobic unit.
The polyols each may be independently a hydrophobic monomer or a hydrophilic monomer, and is preferably a hydrophilic monomer. That is, the repeating unit derived from the polyol may be a hydrophobic unit or a hydrophilic unit, and is preferably a hydrophilic unit.
Preferred conditions for such hydrophobic monomer and hydrophilic monomer, and hydrophobic unit and hydrophilic unit are as described above.

<Core>

The microcapsule according to the embodiment of the present invention encompasses at least one of water or a water-soluble compound as a core.
The water-soluble compound is intended to refer to a compound that can be dissolved in an amount of 10 g or more in 1,000 g of water at room temperature.
The water-soluble compound is preferably a compound different from the hydrophilic monomer.
The water-soluble compound may be solid or liquid at room temperature.
The type of water-soluble compound can be appropriately selected according to the function to be imparted to the microcapsule, and examples thereof include a heat storage material, a bactericide, a deodorant (a compound containing silver ions, or the like), a fragrance (vanillin, or the like), a dye, an agricultural chemical (clothianidin water-soluble powder, or the like), a seasoning, a pharmaceutical ingredient, a cosmetic ingredient, a dye, an adhesive, a curing agent (2-methylimidazole, or the like), and a foaming agent.
In a case where the water-soluble compound is a heat storage material, the heat storage material is preferably, for example, an aliphatic diol, a sugar, a sugar alcohol, or an inorganic salt, and more preferably an aliphatic diol, a sugar alcohol, or an inorganic hydrated salt. Examples of the aliphatic diol include 1,6-hexanediol and 1,8-octanediol. Examples of the sugar and the sugar alcohol include xylitol, erythritol, galactitol, and dihydroxyacetone. Examples of the inorganic hydrated salt include an alkali metal chloride hydrate (for example, a sodium chloride dihydrate), an alkali metal acetate hydrate (for example, a sodium acetate hydrate), an alkali metal sulfate hydrate (for example, a sodium sulfate hydrate), an alkali metal thiosulfate hydrate (for example, a sodium thiosulfate hydrate), an alkaline earth metal sulfate hydrate (for example, a calcium sulfate hydrate), and an alkaline earth metal chloride hydrate (for example, a calcium chloride hydrate).
In a case where the water-soluble compound is a bactericide, the bactericide is preferably, for example, an inorganic salt, and more preferably sodium chlorite, sodium hypochlorite, or the like.
In a case where the water-soluble compound is a dye, the dye is preferably an acid dye or a basic dye known

in the Color Index, more specific examples of which include Acid Blue 9, 45, and 249, Acid Yellow 17, 23, 42, 44, 79, and 142, Acid Red 51, 52, 80, 82, 87, 92, 249, 254, and 388, Acid Violet 9, Acid Black 1, 2, 24, and 94, Basic Yellow 1, 2, 13, 19, 21, 25, 32, 36, 40, and 51, Basic Blue 1, 3, 9, 11, 16, 17, 24, 28, 41, 45, 54, 65, and 66, Basic Red 1, 5, 12, 19, 22, 29, 37, 39, and 92, Basic Violet 10, and Basic Black 2 and 8.

The content of the water-soluble compound is preferably 0.1% to 100% by mass, more preferably 0.1% to 90% by mass, and still more preferably 0.5% to 60% by mass with respect to the total mass of the core.

The content of water is preferably 1% to 100% by mass, more preferably 1% to 99.9% by mass, and still more preferably 40% to 99.5% by mass with respect to the total mass of the core.

For example, the core may contain only water, substantially free of a water-soluble compound. Such a microcapsule can be used, for example, as a potential curing agent in an adhesive that hardens in contact with water. The fact that the core is substantially free of a water-soluble compound is intended, for example, to mean that the content of the water-soluble compound is less than 0.1% by mass with respect to the total mass of the core.

Similarly, the core may contain only a water-soluble compound, substantially free of water. The fact that the core is substantially free of water is intended, for example, to mean that the content of the water is less than 1% by mass with respect to the total mass of the core.

Similarly, the core may contain both water and a water-soluble compound. Such a microcapsule can be applied to, for example, a heat storage capsule that develops heat storage performance in a state where a water-soluble compound is dissolved in water, and a coloring agent capsule that develops color in a microcapsule in a state of being dissolved in water.

The water-soluble compound may be used alone or in combination of two or more thereof.

The total weight of the water and the water-soluble compound is preferably 80% by mass or more, more preferably 90% by mass or more, and particularly preferably 95% by mass or more with respect to the total weight of the core. The upper limit is not particularly limited, and is 100% by mass.

The content (filling rate) of the target ingredient (water and/or water-soluble compound) in the microcapsule according to the embodiment of the present invention with respect to the total solid content of the microcapsule is, for example, preferably 20% by mass or more, more preferably more than 30% by mass, and still more preferably 40% by mass or more. The upper limit of the content (filling rate) is not particularly limited, and is, for example, 90% by mass or less in a case where the target ingredient is only a water-soluble compound, and 2,500% by mass or less in a case where water is included in the target ingredient.

The target ingredient may be one or both of water and a water-soluble compound. In a case where the core contains both water and a water-soluble compound, only one of them may be the target ingredient.

In addition, in the present specification, the solid content is intended to refer to an ingredient other than water contained in the microcapsule, and in a case where it is other than water, such an ingredient is assumed to be a solid content even in a case where the ingredient is liquid.

<Other ingredients>

The microcapsule may contain ingredients other than the foregoing ingredients.

As other ingredients, for example, a particle (for example, a metal-containing particle such as a silver particle), an ultraviolet absorber, a light stabilizer, an antioxidant, a colorant other than a water-soluble compound, and/or a wax may be contained in the core and/or the capsule wall.

In addition, examples of other ingredients include ingredients used for the production of a microcapsule (a surfactant, a dispersant, an initiator and a reactant thereof, a curing accelerator, and unreacted ingredients of hydrophobic monomers and/or hydrophilic monomers). Such ingredients may be intentionally or unavoidably contained in the core and/or the capsule wall.

In addition, as other ingredients, ingredients that are desorbed in a case where the hydrophobic monomer and the hydrophilic monomer are polymerized may be intentionally or unavoidably contained in the core and/or the capsule wall.

In a case where other ingredients are contained in the core, the content of the other ingredients contained in the core is preferably 0% to 20% by mass with respect to the total mass of the core.

In a case where other ingredients are contained in the capsule wall, the content of the other ingredients contained in the capsule wall is preferably 0% to 20% by mass with respect to the total mass of the core.

[Method for producing microcapsule]

The method for producing a microcapsule according to the embodiment of the present invention is not particularly limited, and examples thereof include known methods such as an interfacial polymerization method, an internal polymerization method, a phase separation method, an external polymerization method, and a coacervation method. Of these,

the interfacial polymerization method is preferable.

More specifically, the method for producing a microcapsule according to the embodiment of the present invention is preferably, for example, a method for producing a microcapsule, including a step C of dispersing a "water phase containing water, a hydrophilic monomer added as desired, and a water-soluble compound added as desired" in an "oil phase containing a hydrophobic monomer, a hydrophilic monomer added as desired, and a water-insoluble solvent" to prepare an emulsified liquid (emulsification step), and a step D of polymerizing the hydrophobic monomer and the hydrophilic monomer to form a capsule wall to form a microcapsule encompassing at least water (encapsulation step).

In the method, at least one of the oil phase or the water phase contains a hydrophilic monomer.

In addition, the method may include at least one of a step A of preparing the water phase or a step B of preparing the oil phase before the step C.

Hereinafter, the method will be described in detail.

<Step A and Step B>

The step A and step B may be a combination of a step A1 of preparing a water phase containing a hydrophilic monomer and water with a step B1 of preparing an oil phase containing a hydrophobic monomer and a water-insoluble solvent.

The oil phase in the step B1 may further have a hydrophilic monomer. It is also preferable that the oil phase in the step B1 is substantially free of a hydrophilic monomer. The fact that the oil phase is substantially free of a hydrophilic monomer is intended, for example, to mean that the content of the hydrophilic monomer is less than 0.01% by mass with respect to the total mass of the oil phase.

In addition, the step A and step B may be a combination a step A2 of preparing a water phase containing water with a step B2 of preparing an oil phase containing a hydrophobic monomer, a hydrophilic monomer, and a water-insoluble solvent.

The water phase in the step A2 may further have a hydrophilic monomer. It is also preferable that the water phase in the step A2 is substantially free of a hydrophilic monomer. The fact that the water phase is substantially free of a hydrophilic monomer is intended, for example, to mean that the content of the hydrophilic monomer is less than 0.01% by mass with respect to the total mass of the water phase.

(Water phase)

The water phase contains at least water and, if desired, a water-soluble compound and/or a hydrophilic monomer. That is, the water phase in the step A1 may further contain a water-soluble compound, and the water phase in the step A2 may further contain a water-soluble compound.

The content of water in the water phase is preferably 20% to 100% by mass, more preferably 40% to 100% by mass, and still more preferably 60% to 100% by mass with respect to the total mass of the water phase.

In a case where the water phase contains a water-soluble compound, the content of the water-soluble compound is more preferably 0.1% to 99.9% by mass and more preferably 1% to 60% by mass with respect to the total mass of the water phase. The description of the water-soluble compound is as described above.

The water phase may be substantially free of a water-soluble compound. For example, the content of the water-soluble compound may be less than 0.1% by mass with respect to the total mass of the water phase.

In a case where the water-soluble compound is added to the water phase in the form of a hydrate, the hydrated water in the water-soluble compound is counted as the content of water in the water phase, and is not counted as the content of the water-soluble compound.

In a case where the water phase contains a hydrophilic monomer, the content of the hydrophilic monomer is preferably 0.01% to 30% by mass, more preferably 0.1% to 25% by mass, still more preferably 0.5% to 20% by mass, and particularly preferably 0.8% to 15% by mass with respect to the total mass of the water phase. The description of the hydrophilic monomer is as described above.

In addition, for example, in a case where the specific resin of the capsule wall to be formed is a radical polymer, the water phase preferably contains a water-soluble initiator (for example, a water-soluble azo polymerization initiator, or a water-soluble peroxide such as potassium persulfate), from the viewpoint that a microcapsule satisfying Expression (1) can be easily formed. The content of a water-soluble radical polymerization initiator is preferably 0.0001% to 5% by mass, more preferably 0.01% to 1% by mass, and still more preferably 0.1% to 3% by mass with respect to the total mass of the water phase.

The water phase may contain a water-insoluble particle (for example, a metal-containing particle such as a silver particle). The particle may be present in the water phase, for example, in the form of a colloid.

(Oil phase)

The oil phase contains a hydrophobic monomer, a hydrophilic monomer added as desired, and a water-insoluble solvent.

The water-insoluble solvent in the oil phase is preferably a compound having a soluble amount of less than 90 g (preferably less than 50 g, more preferably less than 10 g) with respect to 1,000 g of water at room temperature and being liquid at room temperature.

Specific examples of the water-insoluble solvent include silicone oil (silicone oil SRX-310 or the like), toluene, hexane, cyclohexanone, glycerol ester oil, animal oil, vegetable oil, and mineral oil.

As the water-insoluble solvent, it is preferable to use a compound that is stable with respect to the hydrophilic monomer and the hydrophobic monomer.

The content of the water-insoluble solvent is preferably 50% to 99.9% by mass, more preferably 80% to 99.5% by mass, and still more preferably 80% to 99% by mass with respect to the total mass of the oil phase.

The content of the hydrophobic monomer is preferably 0.1% to 30% by mass, more preferably 0.5% to 20% by mass, and still more preferably 1% to 15% by mass with respect to the total mass of the oil phase. The description of the hydrophobic monomer is as described above. The hydrophobic monomer is preferably dissolved in a water-insoluble solvent.

In addition, for example, in a case where the resin polymerized in the step D is a radical polymer, the hydrophobic monomer preferably includes the above-mentioned specific hydrophobic monomer.

The content of the specific hydrophobic monomer is preferably 50% to 100% by mass, more preferably 75% to 100% by mass, and still more preferably 95% to 100% by mass with respect to the total mass of the hydrophobic monomer.

In a case where the oil phase contains a hydrophilic monomer, the content of the hydrophilic monomer is preferably 0.01% to 20% by mass, more preferably 0.05% to 10% by mass, and still more preferably 0.1% to 5% by mass with respect to the total mass of the oil phase. The description of the hydrophilic monomer is as described above. The hydrophilic monomer is preferably dissolved in a water-insoluble solvent.

The oil phase may contain an emulsifier. The emulsifier is preferably, for example, an emulsifier having an HLB value of 0 to 10.0 (preferably 0 to 8). In the present specification, the HLB value is obtained by, for example, the Griffin's method.

Examples of the emulsifier include sorbitan monolaurate, sorbitan monopalmitate, sorbitan monostearate, sorbitan distearate, sorbitan monooleate, sorbitan sesquioleate, sorbitan tristearate, and sorbitan trioleate.

As the emulsifier, it is preferable to use a compound that is stable with respect to the hydrophilic monomer and the hydrophobic monomer.

The content of the emulsifier is preferably 0.05% to 15% by mass, more preferably 0.1% to 10% by mass, and still more preferably 0.3% to 5% by mass with respect to the total mass of the oil phase.

In addition, for example, in a case where the resin polymerized in the step D is a radical polymer, the hydrophilic monomer in the water phase or the oil phase preferably includes the above-mentioned specific hydrophilic monomer.

The content of the specific hydrophilic monomer is preferably 50% to 100% by mass, more preferably 75% to 100% by mass, and still more preferably 95% to 100% by mass with respect to the total mass of the hydrophilic monomer.

The water phase and/or the oil phase may contain other ingredients as appropriate, if desired, in addition to the above-mentioned ingredients.

<Step C (emulsification step)>

In the step C, the above-mentioned water phase is dispersed in the above-mentioned oil phase to prepare an emulsified liquid.

In dispersing the water phase in the oil phase, the water phase and the oil phase are mixed.

In a case of mixing the water phase and the oil phase, all of the water phase may be mixed with all of the oil phase at once, or the two phases may be mixed sequentially.

In addition, a mixed liquid in which all or a part of at least a part of the ingredients of the water phase and all or a part of at least a part of the ingredients of the oil phase are mixed is obtained, and before, during, and/or during a treatment of bringing the mixed liquid into an emulsified state, the remaining ingredients of the water phase and/or the oil phase may be mixed with the mixed liquid at once or sequentially.

As a method of dispersing and emulsifying the mixed water phase (all or a part of at least a part of the ingredients of the water phase) and oil phase (all or a part of at least a part of the ingredients of the oil phase), for example, a method using a homogenizer, a manton gaulin, an ultrasonic disperser, a dissolver, a keddy mill, or other known dispersion apparatus can be used.

The mixing ratio of the water phase to the oil phase (that is, the mass of the water phase/the mass of the oil

phase) is preferably 0.01 or more and less than 1.0, more preferably 0.05 to 0.9, and still more preferably 0.1 to 0.5. In a case where the mixing ratio is within the above range, a water-in-oil type emulsified liquid which can be maintained at an appropriate viscosity and has excellent manufacturing suitability and excellent stability can be obtained.

The content of water is preferably 3% to 45% by mass, more preferably 5% to 40% by mass, and still more preferably 8% to 35% by mass with respect to the total mass of the emulsified liquid.

In a case where the emulsified liquid contains a water-soluble compound, the content of the water-soluble compound is preferably 0.01% to 40% by mass, more preferably 0.1% to 30% by mass, and still more preferably 0.3% to 20% by mass with respect to the total mass of the emulsified liquid.

The content of the water-insoluble solvent is preferably 50% to 97% by mass, more preferably 60% to 95% by mass, and still more preferably 70% to 90% by mass with respect to the total mass of the emulsified liquid.

The content of the hydrophilic monomer is preferably 0.005% to 18% by mass, more preferably 0.01% to 8% by mass, and still more preferably 0.05% to 4% by mass with respect to the total mass of the emulsified liquid.

The content of the hydrophobic monomer is preferably 0.05% to 25% by mass, more preferably 0.1% to 18% by mass, and still more preferably 0.5% to 13% by mass with respect to the total mass of the emulsified liquid.

In a case where the emulsified liquid contains a water-soluble radical polymerization initiator, the content of the water-soluble radical polymerization initiator is preferably 0.0005% to 3% by mass, more preferably 0.005% to 2% by mass, and still more preferably 0.02% to 1% by mass with respect to the total mass of the emulsified liquid.

In a case where the emulsified liquid contains an emulsifier, the content of the emulsifier is preferably 0.01% to 12% by mass, more preferably 0.05% to 8% by mass, and still more preferably 0.2% to 4% by mass with respect to the total mass of the emulsified liquid.

The expression "total mass of the emulsified liquid" may be read as the "total mass of the water phase and the oil phase".

In the emulsified liquid (total of the water phase and the oil phase), the content of the hydrophilic monomer is preferably 70% by mass or less, more preferably 40% by mass or less, still more preferably 32% by mass or less, particularly preferably 25% by mass or less, and most preferably 10% by mass or less with respect to the total content of the hydrophilic monomer and the hydrophobic monomer. The lower limit of the content of the hydrophilic unit is, for example, preferably 0.5% by mass or more, more preferably 1% by mass or more, and still more preferably 2% by mass or more.

The content of the hydrophobic monomer is preferably 30% by mass or more, more preferably 60% by mass or more, still more preferably 68% by mass or more, particularly preferably 75% by mass or more, and most preferably 90% by mass or more with respect to the total content of the hydrophilic monomer and the hydrophobic monomer. The upper limit of the content of the hydrophobic unit is, for example, preferably 99.5% by mass or less, more preferably 99% by mass or less, and still more preferably 98% by mass or less.

Above all, in a case where the emulsified liquid contains a hydrophilic radically polymerizable monomer, the content of the hydrophilic radically polymerizable monomer is preferably 25% by mass or less and more preferably 10% by mass or less with respect to the total content of the hydrophilic monomer and the hydrophobic monomer (preferably with respect to the total content of the hydrophilic radically polymerizable monomer and the hydrophobic radically polymerizable monomer). The lower limit of the content of the hydrophilic radically polymerizable monomer is, for example, preferably 0.5% by mass or more, more preferably 1% by mass or more, and still more preferably 2% by mass or more.

In a case where the emulsified liquid contains a hydrophobic radically polymerizable monomer, the content of the hydrophobic radically polymerizable monomer is preferably 75% by mass or more and more preferably 90% by mass or more with respect to the total content of the hydrophilic monomer and the hydrophobic monomer (preferably with respect to the total content of the hydrophilic radically polymerizable monomer and the hydrophobic radically polymerizable monomer). The upper limit of the content of the hydrophobic radically polymerizable monomer is, for example, preferably 99.5% by mass or less, more preferably 99% by mass or less, and still more preferably 98% by mass or less.

The content of the polyfunctional monomer is preferably 5% to 100% by mass, more preferably 10% to 100% by mass, and still more preferably 20% to 100% by mass with respect to the total content of the hydrophilic monomer and the hydrophobic monomer.

The content of the polyfunctional hydrophobic monomer is preferably 5% to 100% by mass, more preferably 10% to 100% by mass, and still more preferably 20% to 100% by mass with respect to the total mass of the hydrophobic monomer.

Above all, in a case where the emulsified liquid contains a polyfunctional radically polymerizable monomer, the content of the polyfunctional radically polymerizable monomer is preferably 20% by mass or more, more preferably 30% by mass or more, still more preferably 30% to 98% by mass, and particularly preferably 30% to 80% by mass with respect to the total content of the hydrophilic monomer and the hydrophobic monomer (preferably with respect to the total content of the hydrophilic radically polymerizable monomer and the hydrophobic radically polymerizable monomer).

In addition, in a case where the emulsified liquid contains a hydrophobic radically polymerizable monomer, it is also preferable that the emulsified liquid contains 19% to 70% by mass of monofunctional (meth)acrylic acid esters

that are hydrophobic monomers having a ClogP value of 1.90 or more, 20% to 80% by mass of polyfunctional (meth)acrylic acid esters that are hydrophobic monomers having a ClogP value of 1.90 or more, and 1% to 10% by mass of (meth)acrylic acid esters that are hydrophilic monomers having a ClogP value of less than 0.70, with respect to the total content of the hydrophilic monomer and the hydrophobic monomer (preferably with respect to the total content of the hydrophilic radically polymerizable monomer and the hydrophobic radically polymerizable monomer).

In the middle of the step C, the polymerization reaction of the hydrophobic monomer and the hydrophilic monomer may partially proceed, either intentionally or unavoidably.

<Step D (encapsulation step)>

The step D is a step of polymerizing a hydrophobic monomer and a hydrophilic monomer to form a capsule wall to form a microcapsule encompassing water.

The polymerization is usually carried out at the interface between the oil phase and the water phase of the hydrophobic monomer and the hydrophilic monomer.

The microcapsule thus obtained encompasses water by the capsule wall. In addition, the microcapsule thus obtained may further contain a water-soluble compound, if desired. In order to encompass the water-soluble compound by the microcapsule, for example, a water phase containing the water-soluble compound may be used.

(Polymerization)

The polymerization is preferably carried out under heating. The reaction temperature in the polymerization varies depending on the combination of a hydrophobic monomer and a hydrophilic monomer, or the like and is, for example, preferably 40°C to 100°C and more preferably 50°C to 80°C.

In addition, the reaction time for polymerization also varies depending on the combination of a hydrophobic monomer and a hydrophilic monomer, or the like and is, for example, preferably 0.5 to 10 hours and more preferably 1 to 5 hours.

A higher polymerization temperature leads to a shorter polymerization time, but in a case where an inclusion or a specific resin that may be decomposed at a high temperature is used, it is desirable to select a polymerization initiator that acts at a low temperature and carry out the polymerization at a relatively low temperature.

In order to prevent the aggregation of microcapsules during the polymerization, it is preferable to further add a water-insoluble solvent to reduce the probability of collision between the microcapsules during the polymerization, and it is also preferable to carry out sufficient stirring.

A dispersant or the like for preventing aggregation may be added again during the polymerization.

The microcapsule obtained through the step D may be further subjected to a drying treatment or the like to obtain a microcapsule in which the amount of water contained in the core is reduced, or a microcapsule in which the core is substantially free of water.

Examples

Hereinafter, the present invention will be described in more detail with reference to Examples. The present invention is not limited to the following examples as long as the gist thereof is not exceeded.

[Preparation of microcapsule]

<Monomers>

The monomers used for the preparation of the microcapsule are shown. The value in parentheses is the ClogP value of the monomer.

(Hydrophilic monomer)

- AM: acrylamide (-0.61)
- AA: acrylic acid (0.35)
- MAA: methacrylic acid (0.66)
- HEA: 2-hydroxyethyl acrylate (-0.01)
- HEMA: 2-hydroxyethyl methacrylate (0.30)

- EDA: ethylenediamine (-2.02)
- DETA: diethylenetriamine (-2.27)

(Hydrophobic monomer (non-polyfunctional radically polymerizable monomer))

- MA: methyl acrylate (0.80)
- MMA: methyl methacrylate (1.11)
- BA: butyl acrylate (2.38)
- BZMA: benzyl methacrylate (2.87)
- HA: hexyl acrylate (3.44)

(Hydrophobic monomer (polyfunctional radically polymerizable monomer))

- NDMA: 1,9-nonanediol dimethacrylate (5.22)
- TMPTMA: trimethylolpropane trimethacrylate (4.31)

(Hydrophobic monomer (polyfunctional isocyanate))

- D750: BURNOCK D750 (trimethylolpropane adduct of toluene diisocyanate) (6.80)

(Hydrophobic monomer (non-polyfunctional acid chloride))

- TPC: terephthalic acid chloride (0.99)

<Synthesis>

(Example 1)

Silicone oil SRX-310 (40 g) was placed in a three-neck flask having a capacity of 100 mL, and butyl acrylate (1.2 g) and benzyl methacrylate (2.2 g) were dissolved in the silicone oil SRX-310 to prepare an oil phase.
Calcium chloride (3 g), methacrylic acid (0.6 g), and a water-soluble azo polymerization initiator V-50 (0.05 g) were dissolved in water (7 g) to prepare a water phase.
The water phase was added to the oil phase, followed by stirring at 180 rpm for 1 hour at room temperature under a nitrogen stream to obtain an emulsified mixed liquid. The mixed liquid was heated to an internal temperature of 70°C, stirred at 180 rpm for 3 hours at the same temperature, and then cooled to room temperature, and hexane (50 mL) was added thereto. The mixed liquid was filtered to obtain white particles (microcapsules encompassing a 30% by mass calcium chloride aqueous solution) as a filtrate.

(Example 2)

Calcium chloride (3 g) was dissolved in water (7 g), and ethylenediamine (0.91 g) was further added to the water to prepare a water phase.
Toluene (30 mL) and sorbitan trioleate (0.4 g) as a surfactant were placed in a SUS container having a capacity of 200 mL.
The water phase was further added to the SUS container, followed by stirring at 2,500 rpm for 5 minutes using an EXCEL AUTO HOMOGENIZER (available from Nihonseiki Kaisha Ltd.) to obtain an emulsified mixed liquid.
Further, a suspension of terephthalic acid chloride (3.08 g) suspended in toluene (10 mL) was added dropwise over 5 minutes while the mixed liquid was being stirred at 2,500 rpm. After the dropwise addition of the suspension to the mixed liquid was completed, the mixed liquid was stirred at 2,500 rpm for 1 minute, transferred to a three-neck flask having a capacity of 100 mL, stirred at 200 rpm, and heated to 70°C. The mixed liquid was stirred at the same temperature

at 200 rpm for 2 hours, then the mixed liquid was cooled to room temperature, stirring was stopped, and the mixed liquid was allowed to stand for 30 minutes. Then, the supernatant of the mixed liquid was discarded, and toluene for the discarded supernatant was added to obtain a dispersion liquid. The dispersion liquid was filtered to obtain white particles (microcapsules encompassing a 30% by mass calcium chloride aqueous solution) as a filtrate.

(Example 3)

Calcium chloride (3 g) was dissolved in water (7 g), and diethylenetriamine (0.2 g) was further added to the water to prepare a water phase.

Toluene (30 mL) and sorbitan trioleate (0.4 g) as a surfactant were added and placed in a SUS container having a capacity of 200 mL.

The water phase was further added to the SUS container, followed by stirring at 4,000 rpm for 5 minutes using an EXCEL AUTO HOMOGENIZER (available from Nihonseiki Kaisha Ltd.) to obtain an emulsified mixed liquid.

Further, a dissolved liquid prepared by dissolving BURNOCK D-750 (available from DIC Corporation, trimethylolpropane-modified product of toluene diisocyanate) (2.0 g) in toluene (6 mL) was added dropwise to the mixed liquid over 5 minutes while the mixed liquid was being stirred at 4,000 rpm. After the dropwise addition of the dissolved liquid to the mixed liquid was completed, the mixed liquid was stirred at 4,000 rpm for 1 minute, transferred to a three-neck flask having a capacity of 100 mL, stirred at 200 rpm, and heated to 60°C. The mixed liquid was stirred at the same temperature at 200 rpm for 2 hours, then the mixed liquid was cooled to room temperature, stirring was stopped, and the mixed liquid was allowed to stand for 30 minutes. Then, the supernatant of the mixed liquid was discarded, and toluene for the discarded supernatant was added to obtain a dispersion liquid. The dispersion liquid was filtered to obtain white particles (microcapsules encompassing a 30% by mass calcium chloride aqueous solution) as a filtrate.

(Examples 4 to 11)

Particles (microcapsules) were obtained in the same manner as in Example 1, except that the amount and type of monomer, and the type of initiator were changed as shown in the table which will be given later.

(Example 12)

Silicone oil SRX-310 (40 g) was placed in a three-neck flask having a capacity of 100 mL, and benzyl methacrylate (0.90 g) and 1,9-nonanediol dimethacrylate (2.96 g) were dissolved in the silicone oil SRX-310 to prepare an oil phase.

Calcium chloride (3 g), methacrylic acid (0.13 g), and potassium persulfate (0.05 g) were dissolved in water (7 g) to prepare a water phase.

The water phase was added to the oil phase, followed by stirring at 250 rpm for 1 hour at room temperature under a nitrogen stream to obtain an emulsified mixed liquid. The mixed liquid was heated to an internal temperature of 70°C, stirred at 250 rpm for 3 hours at the same temperature, and then cooled to room temperature, and hexane (50 mL) was added thereto. The mixed liquid was filtered to obtain white particles (microcapsules encompassing a 30% by mass calcium chloride aqueous solution) as a filtrate.

(Example 13)

Particles (microcapsules encompassing a calcium chloride aqueous solution) were obtained in the same manner as in Example 1, except that the amount and type of monomer, and the type of initiator were changed as shown in the table which will be given later.

(Example 14)

Silicone oil SRX-310 (40 g) was placed in a three-neck flask having a capacity of 100 mL, and benzyl methacrylate (0.50 g) and 1,9-nonanediol dimethacrylate (1.42 g) were dissolved in the silicone oil SRX-310 to prepare an oil phase.

2-methylimidazole (2 g), 2-hydroxyethyl methacrylate (0.08 g), and potassium persulfate (0.05 g) were dissolved in water (8 g) to prepare a water phase.

The water phase was added to the oil phase, followed by stirring at 250 rpm for 1 hour at room temperature under a nitrogen stream to obtain an emulsified mixed liquid. The mixed liquid was heated to an internal temperature of 70°C, stirred at 250 rpm for 3 hours at the same temperature, and then cooled to room temperature, and hexane (50 mL) was added thereto. The mixed liquid was filtered to obtain white particles (microcapsules encompassing a 20% by mass 2-methylimidazole aqueous solution) as a filtrate.

(Example 14-2)

The particles (microcapsules encompassing a 2-methylimidazole aqueous solution) obtained in Example 14 were further dried at 50°C for 3 days using a vacuum dryer to obtain particles (microcapsules encompassing 2-methylimidazole) that have been subjected to a drying treatment.

(Example 15)

The procedure was carried out in the same manner as in Example 12, except that the combination of calcium chloride (3 g) and water (7 g) was changed to a combination of sodium acetate trihydrate (5.99 g), calcium carbonate (0.01 g), and water (4 g).
Further, the obtained white particles were dried at 50°C for 12 hours and then allowed to stand at -40°C for 24 hours to obtain microcapsules encompassing a sodium acetate trihydrate.
The average particle diameter of the obtained white particles (particles before drying) was 100 μm, and in a case where the cross section was observed by SEM, the film thickness was about 9 μm and δ/Dm was 0.09.

(Example 16)

The procedure was carried out in the same manner as in Example 3, except that the combination of calcium chloride (3 g) and water (7 g) was changed to a 1% by mass vanillin aqueous solution (10 g), whereby microcapsules encompassing a vanillin aqueous solution were obtained.
Vanillin is a compound that can be used, for example, as a fragrance.

(Example 17)

The procedure was carried out in the same manner as in Example 12, except that the combination of calcium chloride (3 g) and water (7 g) was changed to a silver nanocolloidal solution (NANOPURE S-01, pure silver nanocolloidal stock solution manufactured by JAPAN ION Corporation) (10 g), whereby microcapsules encompassing a silver nanocolloidal solution were obtained.
The silver nanocolloidal solution can be used as a deodorant, for example.

(Example 18)

The procedure was carried out in the same manner as in Example 12, except that calcium chloride (3 g) was changed to Acid Blue 9 (manufactured by Tokyo Chemical Industry Co., Ltd.) that is a dye (3 g), whereby blue microcapsules encompassing an Acid Blue 9 aqueous solution were obtained.

(Example 19)

The procedure was carried out in the same manner as in Example 12, except that the combination of calcium chloride (3 g) and water (7 g) was changed to a 1,000-fold diluted clothianidin water-soluble powder (Dantotsu (R) water-soluble powder) (10 g), whereby microcapsules encompassing an aqueous solution of clothianidin were obtained.
The clothianidin water-soluble powder can be used as an agricultural chemical, for example.

(Example 20)

The procedure was carried out in the same manner as in Example 12, except that the combination of calcium chloride (3 g) and water (7 g) was changed to liquid fertilizer (PHOSPLUS, manufactured by OAT Agrio Co., Ltd.) (10 g), whereby microcapsules encompassing liquid fertilizer were obtained.

(Example 21)

The procedure was carried out in the same manner as in Example 3, except that calcium chloride (3 g) was changed to sodium chlorite (3 g), whereby microcapsules encompassing a sodium chlorite aqueous solution were obtained.
Sodium chlorite is a compound that can be used, for example, as a bactericide or a precursor thereof.

(Example 22)

The procedure was carried out in the same manner as in Example 12, except that the combination of calcium chloride (3 g) and water (7 g) was changed to a combination of sodium hypochlorite (1 g) and water (9 g), whereby microcapsules encompassing a sodium hypochlorite aqueous solution were obtained.
Sodium chlorite is a compound that can be used, for example, as a bactericide.

(Example 23)

The procedure was carried out in the same manner as in Claim 8, except that the combination of calcium chloride (3 g) and water (7 g) was changed to water (10 g), whereby microcapsules encompassing water were obtained.

(Example 24)

The procedure was carried out in the same manner as in Example 12, except that the combination of calcium chloride (3 g) and water (7 g) was changed to soy sauce (10 g), whereby microcapsules encompassing soy sauce were obtained.

(Comparative Example 1 (example using only hydrophobic monomer))

The procedure was carried out in the same manner as in Example 12, except that the combination of benzyl methacrylate (0.90 g) and 1,9-nonanediol dimethacrylate (2.96 g) was changed to a combination of butyl acrylate (0.96 g), benzyl methacrylate (1.74 g), and 1,9-nonanediol dimethacrylate (1.30 g), and methacrylic acid was not added to the water phase. As a result, the particles were crushed during filtration, and therefore microcapsules encompassing a calcium chloride aqueous solution could not be obtained.

(Comparative Example 2 (example using only hydrophilic monomer))

The procedure was carried out in the same manner as in Example 12, except that benzyl methacrylate (0.90 g) and 1,9-nonanediol dimethacrylate (2.96 g) were not used and the amount of methacrylic acid in the water phase was increased to 4 g, whereby white particles were obtained.
In a case where the cross section of the obtained particles was observed by SEM, it was confirmed that the obtained particles had no wall, the resin was packed even inside the particles, and the inclusions were only present in the gaps of the resin.
A micrograph of the cross section of the microcapsule in Example 12 is shown as Fig. 1, and a micrograph of the cross section of the microcapsule in Comparative Example 2 is shown as Fig. 2.

(Comparative Example 3 (example using resin consisting only of hydrophilic monomer))

An acrylic acid-acrylamide copolymer (molecular weight: 4,000) (0.7 g) and 2-methylimidazole (0.3 g) were dissolved in water (22.5 g) to obtain a water phase.
An oil phase was obtained by mixing sorbitan sesquioleate as a surfactant in an isoparaffin-based solvent (ISOPAR M) so as to be 1% by mass.
The water phase (23.5 g) was added to the oil phase (112.5 g), followed by stirring at 5,000 rpm for 10 minutes using an EXCEL AUTO HOMOGENIZER (available from Nihonseiki Kaisha Ltd.) to obtain an emulsified and dispersed emulsified liquid.
The obtained emulsified liquid was heated in a reactor equipped with a decompression device under the conditions of 70°C and 0.1 MPa, and the pressure was reduced to distill off water to obtain a dispersion liquid of microcapsules.
The obtained dispersion liquid was repeatedly washed with cyclohexane and then dried at 50°C for 3 days using a vacuum dryer to obtain microcapsules.

[Evaluation]

The microcapsules obtained in the foregoing Examples or Comparative Examples were evaluated.

<Evaluation of particle shape, median diameter (average particle diameter), and number average wall thickness>

The volume-based median diameter of the particles (microcapsules) of Examples and Comparative Examples

was measured using MICROTRAC MT3300EXII (manufactured by Nikkiso Co., Ltd.).

In addition, using SEM, the number average wall thickness of the particles (microcapsules) of Examples and Comparative Examples was determined by the method described in the specification.

It was confirmed that all the microcapsules of Examples satisfied "$\delta/Dm \leq 0.40$" ($\delta$: number average wall thickness ($\mu$m) of microcapsules, Dm: volume-based median diameter ($\mu$m) of microcapsules).

In addition, upon observing the cross section of the microcapsules using SEM, it was confirmed that all the microcapsules of Examples were mononuclear microcapsules.

In addition, it was confirmed that the microcapsules of Comparative Example 2 were not mononuclear microcapsules, and it was confirmed that the microcapsules of Comparative Example 3 were mononuclear microcapsules.

The median diameter, number average wall thickness, and $\delta$/Dm of the microcapsules of Examples 1 to 15 and Comparative Examples 2 and 3 are shown in the table which will be given later.

The microcapsules of Example 14-2 had a median diameter of 50 $\mu$m, a number average film thickness of 5 $\mu$m, and $\delta$/Dm of 0.10.

In addition, the microcapsules of Example 18 had a median diameter of 110 $\mu$m, a number average film thickness of 8 $\mu$m, and a $\delta$/Dm of 0.07.

<Evaluation of leakage resistance>

1 g of microcapsules was spread out in a circle having a diameter of 2 cm on a hard filter paper (No. 4) placed on a petri dish which was then covered with a lid and allowed to stand at room temperature for 12 hours.

Thereafter, the wetting state of the hard filter paper was classified into the following A to D, and the leakage resistance of the microcapsules was evaluated. In a case where it is rated as A to C, the leakage resistance is good.

A: It cannot be confirmed that it is wet.
B: It cannot be visually confirmed that it is wet, but it can be confirmed that it is slightly moist by touching with hands.
C: It can be visually confirmed that it is wet, but the wet area is narrow (the area where it can be visually confirmed that it is wet is less than 3 cm in diameter).
D: It can be visually confirmed that it is wet, and the wet area is wide (the area where it can be visually confirmed that it is wet is 3 cm or more in diameter).

<Evaluation of morphological stability>

The microcapsules were allowed to stand for 48 hours under the conditions of a temperature of 30°C and a humidity of 90%. Then, the microcapsules were observed with an optical microscope and classified into the following A to D to evaluate the morphological stability of the microcapsules.

A: All microcapsules maintained their shape.
B: The number of microcapsules that were out of shape or had torn capsules and leaked contents was more than 0% and 10% or less of the total.
C: The number of microcapsules that were out of shape or had torn capsules and leaked contents was more than 10% and 30% or less of the total.
D: The number of microcapsules that were out of shape or had torn capsules and leaked contents was more than 30% of the total.

<Evaluation of solvent resistance>

0.5 g of microcapsules encompassing a dye was suspended in 100 ml of toluene to obtain a suspension. The suspension was stirred for 1 hour and then allowed to stand to examine the coloring of the supernatant solution.

In a case where coloring was not confirmed or almost not confirmed in the supernatant solution, it was rated as A, and in a case where clear coloring was confirmed in the supernatant solution, it was rated as B.

In a case where it is rated as A, it can be determined that the microcapsules have good solvent resistance.

[Result]

The table which will be given later shows the characteristics and evaluation results of the microcapsules of each of Examples or Comparative Examples.

In Table 1, the column of "Type of wall" shows the type of resin constituting the capsule wall in the microcapsules prepared in each of Examples.

The column of "Initiator" shows an initiator used in the synthesis of the resin constituting the capsule wall of the microcapsules prepared in each of Examples. The description of "V-50" means a water-soluble azo polymerization initiator V-50 (manufactured by FUJIFILM Wako Pure Chemical Corporation), the description of "VA-57" means a water-soluble azo polymerization initiator VA-057 (manufactured by FUJIFILM Wako Pure Chemical Corporation), and the description of "K persulfate" means potassium persulfate.

The column of "Type of core" shows an ingredient contained as the core in the microcapsules prepared in each of Examples.

The column of "Amount of core (g)" shows a total mass of the core (water and water-soluble compound) in the total mass of the microcapsule prepared in each of Examples.

The column of "Amount of wall (g)" shows a total mass of the capsule wall (resin) in the total mass of the microcapsule prepared in each of Examples.

The column of "Filling rate (% by mass)" shows a content (% by mass) of the water-soluble compound with respect to the total solid content of the microcapsule prepared in each of Examples. In Table 1, the filling rate is shown with the ingredient other than water in the core as the target ingredient. In addition, in a case where the microcapsule is not a mononuclear microcapsule, it is described as "-".

In Example 15, the "type of core", "amount of core (g)", "amount of wall (g)", and "filling rate (% by mass)" of the microcapsules before drying are shown.

Table 2 shows the preparation ratio (mass ratio) of the monomers used for the polymerization of the capsule wall of the microcapsules prepared in each of Examples to the reaction system.

In each of Examples, by measuring the amount of residual monomers by high performance liquid chromatography (HPLC), it was confirmed that substantially the entire amount of the charged monomers was polymerized to form the capsule wall of the microcapsules, and substantially the entire amounts of the charged water and water-soluble compound were incorporated into the microcapsules as the core.

In addition, in a case where the microcapsules of Example 2 were produced, the HCl (hydrogen atom and chlorine atom) desorbed by the reaction between terephthalic acid chloride and ethylenediamine was incorporated into the core in the microcapsules.

In Table 3, the column of "Dm (μm)" shows a volume-based median diameter (μm) of the microcapsules. Comparative Example 1 in which the microcapsules could not be taken out was described as "-".

The column of "δ (μm)" shows a number average wall thickness (μm) of the microcapsules. The microcapsules of Comparative Example 2, which are multinucleated microcapsules and in which the wall thickness cannot be considered, were described as "-". In addition, Comparative Example 1 in which the microcapsules could not be taken out was also described as "-".

The column of "δ/Dm" shows a ratio of the number average wall thickness of the microcapsules to the volume-based median diameter of the microcapsules.

In Example 15, "Dm (μm)", "δ (μm)", and "δ/Dm" of the microcapsules before drying are shown.

In Table 4, the column of "Amount of hydrophilic unit (% by mass)" shows a content (% by mass) of a repeating unit derived from a hydrophilic monomer with respect to the total mass of the resin constituting the capsule wall.

The column of "Hydrophobic polyfunctional" shows whether or not the resin constituting the capsule wall has a repeating unit derived from a polyfunctional hydrophobic polymer. In a case where this requirement is satisfied, it is given as "A", and in a case where this requirement is not satisfied, it is given as "B".

Since the shape of the microcapsules of Comparative Example 1 had collapsed from the beginning, the "morphological stability" was described as "-".

The description of "-" in the column of "Solvent resistance" shows that the microcapsules of the Example have not been subjected to a solvent resistance test.

[Table 1]

| | | Type of wall | Initiator | Type of core | Amountof core (g) | Amount of wall (g) | Filling rate (% by mass) |
|---|---|---|---|---|---|---|---|
| Example | 1 | Acrylic | V-50 | Calcium chloride aqueous solution | 10 | 4 | 42.9 |
| | 2 | Polyamide | - | Calcium chloride aqueous solution | 11.1 | 2.9 | 50.8 |
| | 3 | Polyurethane urea | - | Calcium chloride aqueous solution | 10 | 2.2 | 57.7 |
| | 4 | Acrylic | VA-57 | Calcium chloride aqueous solution | 10 | 4 | 42.9 |
| | 5 | Acrylic | K persulfate | Calcium chloride aqueous solution | 10 | 4 | 42.9 |
| | 6 | Acrylic | K persulfate | Calcium chloride aqueous solution | 10 | 4 | 42.9 |
| | 7 | Acrylic | K persulfate | Calcium chloride aqueous solution | 10 | 4 | 42.9 |
| | 8 | Acrylic | K persulfate | Calcium chloride aqueous solution | 10 | 9 | 25.0 |
| | 9 | Acrylic | K persulfate | Calcium chloride aqueous solution | 10 | 2 | 60.0 |
| | 10 | Acrylic | K persulfate | Calcium chloride aqueous solution | 10 | 6 | 33.3 |
| | 11 | Acrylic | K persulfate | Calcium chloride aqueous solution | 10 | 4 | 42.9 |
| | 12 | Acrylic | K persulfate | Calcium chloride aqueous solution | 10 | 4 | 42.9 |
| | 13 | Acrylic | K persulfate | Calcium chloride aqueous solution | 10 | 4 | 42.9 |
| | 14 | Acrylic | K persulfate | 2-methylimidazole aqueous solution | 10 | 2 | 50.0 |
| | 14-2 | Acrylic | K persulfate | 2-methylimidazole | 2 | 2 | 50.0 |
| | 15 | Acrylic | K persulfate | Sodium acetate aqueous solution | 10 | 4 | 47.5 |
| Comparative Example | 1 | Acrylic | K persulfate | Calcium chloride aqueous solution | 10 | 4 | - |
| | 2 | Acrylic | K persulfate | Calcium chloride aqueous solution | 10 | 4 | - |
| | 3 | Acrylic | - | 2-methylimidazole aqueous solution | 22.8 | 0.7 | 30.0 |

[Table 2]

| | | Monomers that form capsule wall | | | | | | | | | | | | | |
|---|---|---|---|---|---|---|---|---|---|---|---|---|---|---|---|
| | | Hydrophilic monomer | | | | | | Hydrophobic monomer | | | | | | | |
| | | | | | | | | Polyfunctional isocyanate | Non-polyfunctional acid chloride | Non-polyfunctional radically polymerizable monomer | | | | Polyfunctional radically polymerizable monomer | |
| Type | | EDA | DETA | HEA | HEMA | AA | MAA | D750 | TPC | MMA | BA | BZMA | HA | NDMA | TMPTMA |
| ClogP value | | -2.02 | -2.27 | -0.01 | 0.30 | 0.35 | 0.66 | 6.80 | 0.99 | 1.11 | 2.38 | 2.87 | 3.44 | 5.22 | 4.31 |
| Example | 1 | | | | | | 15.0 | | | | 30.0 | 55.0 | | | |
| | 2 | 22.8 | | | | | | | 77.2 | | | | | | |
| | 3 | | 9.1 | | | | | 90.9 | | | | | | | |
| | 4 | | | | 20.0 | | | | | 20.0 | | | 60.0 | | |
| | 5 | | | | | 20.0 | | | | | | 80.0 | | | |
| | 6 | | | | 7.0 | | | | | | | 93.0 | | | |
| | 7 | | | | 7.0 | | | | | | | 60.0 | | 33.0 | |
| | 8 | | | | 4.0 | | | | | | | 60.0 | | 36.0 | |
| | 9 | | | | 4.0 | | | | | | | 25.0 | | 71.0 | |
| | 10 | | | | 7.0 | | | | | | | 60.0 | | 33.0 | |
| | 11 | | | | 7.0 | | | | | | | 25.0 | | 68.0 | |
| | 12 | | | | | | 3.3 | | | | | 22.6 | | 74.1 | |
| | 13 | | | 6.0 | | | | | | | | 25.0 | 25.0 | | 44.0 |
| | 14 | | | | 4.0 | | | | | | | 25.0 | | 71.0 | |
| | 14-2 | | | | 4.0 | | | | | | | 25.0 | | 71.0 | |
| | 15 | | | | | | 3.3 | | | | | 22.6 | | 74.1 | |
| Comparative. Example | 1 | | | | | | | | | | 24.0 | 43.5 | | 32.5 | |
| | 2 | | | | | | 100.0 | | | | | | | | |
| | 3 | Acrylic acid-acrylamide copolymer | | | | | | | | | | | | | |

[Table 3]

| | | Physical properties of microcapsule | | |
|---|---|---|---|---|
| | | Dm (μm) | δ (μm) | δ/Dm |
| Example | 1 | 180 | 30 | 0.17 |
| | 2 | 28 | 3 | 0.11 |
| | 3 | 15 | 0.4 | 0.03 |
| | 4 | 110 | 30 | 0.27 |
| | 5 | 80 | 20 | 0.25 |
| | 6 | 90 | 15 | 0.17 |
| | 7 | 90 | 10 | 0.11 |
| | 8 | 90 | 20 | 0.22 |
| | 9 | 90 | 5 | 0.06 |
| | 10 | 30 | 10 | 0.33 |
| | 11 | 150 | 10 | 0.07 |
| | 12 | 110 | 8 | 0.07 |
| | 13 | 90 | 7 | 0.08 |
| | 14 | 90 | 5 | 0.06 |
| | 14-2 | 50 | 5 | 0.10 |
| | 15 | 100 | 9 | 0.09 |
| Comparative Example | 1 | - | - | - |
| | 2 | 110 | - | - |
| | 3 | 1.5 | 0.55 | 0.37 |

[Table 4]

| | Characteristics of capsule wall | | Results | | |
|---|---|---|---|---|---|
| | Amount of hydrophilic unit (% by mass) | Hydrophobic polyfunctional | Leakage resistance | Morphological stability | Solvent resistance |
| Example 1 | 15.0 | B | B | B | - |
| Example 2 | 30.5 | B | B | B | - |
| Example 3 | 9.1 | A | A | A | - |
| Example 4 | 20.0 | B | B | B | - |
| Example 5 | 20.0 | B | B | B | - |
| Example 6 | 7.0 | B | A | B | - |
| Example 7 | 7.0 | A | A | A | - |
| Example 8 | 4.0 | A | A | A | - |
| Example 9 | 4.0 | A | A | A | - |
| Example 10 | 7.0 | A | A | A | - |
| Example 11 | 7.0 | A | A | A | - |
| Example 12 | 3.3 | A | A | A | - |

(continued)

| | Characteristics of capsule wall | | Results | | |
|---|---|---|---|---|---|
| | Amount of hydrophilic unit (% by mass) | Hydrophobic polyfunctional | Leakage resistance | Morphological stability | Solvent resistance |
| Example 13 | 6.0 | A | A | A | - |
| Example 14 | 4.0 | A | A | A | - |
| Example 18 | 3.0 | A | A | A | A |
| Comparative Example 1 | 0.0 | A | D | - | - |
| Comparative Example 2 | 100.0 | A | D | D | - |
| Comparative Example 3 | - | - | A | D | - |

From the results shown in the tables, it was confirmed that the microcapsules according to the embodiment of the present invention have excellent leakage resistance and morphological stability.

In addition, it was confirmed that the microcapsules according to the embodiment of the present invention have good solvent resistance.

It was confirmed that the leakage resistance is more excellent in a case where the content of the repeating unit derived from the hydrophilic monomer is 32% by mass or less (more preferably 10% by mass or less) with respect to all the repeating units of the resin (See the results of Examples 1, 2, 4, and 5, and the like).

It was confirmed that the morphological stability is more excellent in a case where the hydrophobic monomer includes a polyfunctional hydrophobic monomer (see comparison of the results of Examples 6, 7, and 11, and the like).

**Claims**

**1.** A microcapsule encompassing at least one of water or a water-soluble compound,

wherein a capsule wall of the microcapsule contains a resin having a repeating unit derived from a hydrophobic monomer having a ClogP value of 0.70 or more and a repeating unit derived from a hydrophilic monomer having a ClogP value of less than 0.70, and
the microcapsule satisfies a relationship of Expression (1),

$$\text{Expression (1)} \qquad \delta/\mathrm{Dm} \leq 0.40$$

$\delta$ represents a number average wall thickness ($\mu$m) of the microcapsule, and Dm represents a volume-based median diameter ($\mu$m) of the microcapsule.

**2.** The microcapsule according to claim 1,
wherein the median diameter is 7 to 200 $\mu$m.

**3.** The microcapsule according to claim 1 or 2,
wherein a content of the repeating unit derived from the hydrophilic monomer is 32% by mass or less with respect to all the repeating units of the resin.

**4.** The microcapsule according to any one of claims 1 to 3,
wherein the hydrophobic monomer includes a polyfunctional hydrophobic monomer.

**5.** The microcapsule according to any one of claims 1 to 4,
wherein the hydrophilic monomer includes at least one monomer selected from the group consisting of a (meth)acrylic

acid, a (meth)acrylic acid ester having a ClogP value of less than 0.70, and a (meth)acrylic acid amide having a ClogP value of less than 0.70.

**6.** The microcapsule according to any one of claims 1 to 5,
wherein the hydrophobic monomer includes at least one monomer selected from the group consisting of a (meth)acrylic acid ester having a ClogP value of 0.70 or more and a styrene having a ClogP value of 0.70 or more.

**7.** The microcapsule according to any one of claims 1 to 6,
wherein the hydrophobic monomer includes a (meth)acrylic acid ester having a ClogP value of 1.90 or more.

**8.** A method for producing the microcapsule according to any one of claims 1 to 7, the method comprising:

a step A1 of preparing a water phase containing the hydrophilic monomer and water;
a step B1 of preparing an oil phase containing the hydrophobic monomer and a water-insoluble solvent;
a step C of dispersing the water phase in the oil phase to prepare an emulsified liquid; and
a step D of polymerizing the hydrophobic monomer and the hydrophilic monomer to form the capsule wall to form the microcapsule encompassing the water.

**9.** A method for producing the microcapsule according to any one of claims 1 to 7, the method comprising:

a step A2 of preparing a water phase containing water;
a step B2 of preparing an oil phase containing the hydrophobic monomer, the hydrophilic monomer, and a water-insoluble solvent;
a step C of dispersing the water phase in the oil phase to prepare an emulsified liquid; and
a step D of polymerizing the hydrophobic monomer and the hydrophilic monomer to form the capsule wall to form the microcapsule encompassing the water.

**10.** The method for producing the microcapsule according to claim 8 or 9,

wherein the water phase further contains the water-soluble compound, and
the step D forms the microcapsule encompassing the water and the water-soluble compound.

**11.** The method for producing the microcapsule according to any one of claims 8 to 10, wherein a content of the hydrophilic monomer in the emulsified liquid is 1% to 25% by mass with respect to a total content of the hydrophobic monomer and the hydrophilic monomer.

FIG. 1

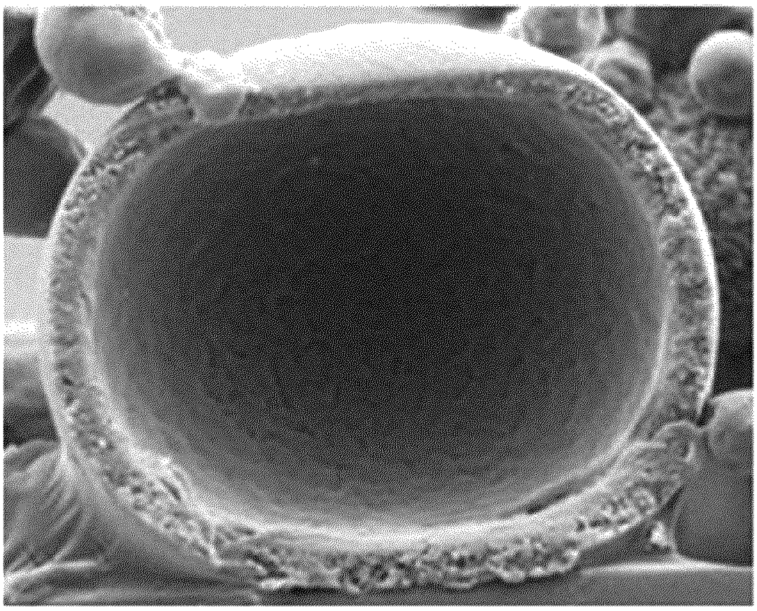

FIG. 2

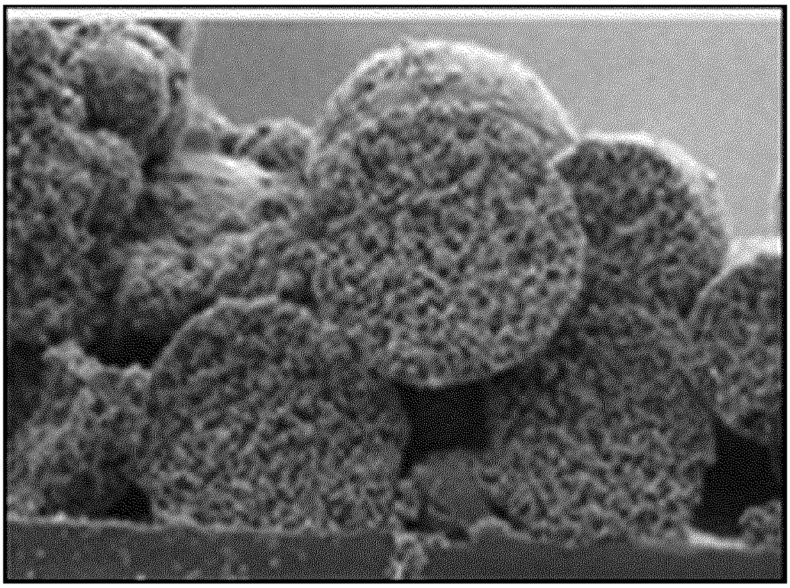

**INTERNATIONAL SEARCH REPORT**

International application No.
PCT/JP2020/034221

A. CLASSIFICATION OF SUBJECT MATTER
Int.Cl. B01J13/16(2006.01)i
FI: B01J13/16

According to International Patent Classification (IPC) or to both national classification and IPC

B. FIELDS SEARCHED

Minimum documentation searched (classification system followed by classification symbols)
Int.Cl. B01J13/02-13/22

Documentation searched other than minimum documentation to the extent that such documents are included in the fields searched

| | |
|---|---|
| Published examined utility model applications of Japan | 1922-1996 |
| Published unexamined utility model applications of Japan | 1971-2020 |
| Registered utility model specifications of Japan | 1996-2020 |
| Published registered utility model applications of Japan | 1994-2020 |

Electronic data base consulted during the international search (name of data base and, where practicable, search terms used)
CAplus/REGISTRY(STN)

C. DOCUMENTS CONSIDERED TO BE RELEVANT

| Category* | Citation of document, with indication, where appropriate, of the relevant passages | Relevant to claim No. |
|---|---|---|
| A | US 2012/0128978 A1 (BALLWEG, T.) 24 May 2012 (2012-05-24), claim 1, example 1 | 1-11 |
| A | JP 2017-518249 A (DOW AGRO SCIENCE LLC) 06 July 2017 (2017-07-06), claim 1, paragraphs [0074]-[0078] | 1-11 |
| A | JP 2015-507529 A (BASF SE) 12 March 2015 (2015-03-12), claim 1, examples 1-14 | 1-11 |
| A | JP 2015-164722 A (SEKISUI CHEMICAL CO., LTD.) 17 September 2015 (2015-09-17), claim 1, examples 1-9 | 1-11 |
| A | WU, D. et al., Aqueous-core capsules via interfacial free radical alternating copolymerization, Macromolecules, 22 July 2006, vol. 39, no. 17, pp. 5848-5853, ISSN 1520-5835, abstract, fig. 1, 6, 7 | 1-11 |

☐ Further documents are listed in the continuation of Box C.

☒ See patent family annex.

* Special categories of cited documents:
"A" document defining the general state of the art which is not considered to be of particular relevance
"E" earlier application or patent but published on or after the international filing date
"L" document which may throw doubts on priority claim(s) or which is cited to establish the publication date of another citation or other special reason (as specified)
"O" document referring to an oral disclosure, use, exhibition or other means
"P" document published prior to the international filing date but later than the priority date claimed

"T" later document published after the international filing date or priority date and not in conflict with the application but cited to understand the principle or theory underlying the invention
"X" document of particular relevance; the claimed invention cannot be considered novel or cannot be considered to involve an inventive step when the document is taken alone
"Y" document of particular relevance; the claimed invention cannot be considered to involve an inventive step when the document is combined with one or more other such documents, such combination being obvious to a person skilled in the art
"&" document member of the same patent family

| Date of the actual completion of the international search | Date of mailing of the international search report |
|---|---|
| 10 November 2020 | 24 November 2020 |

| Name and mailing address of the ISA/ | Authorized officer |
|---|---|
| Japan Patent Office<br>3-4-3, Kasumigaseki, Chiyoda-ku,<br>Tokyo 100-8915, Japan | Telephone No. |

Form PCT/ISA/210 (second sheet) (January 2015)

**INTERNATIONAL SEARCH REPORT**
Information on patent family members

International application No.
PCT/JP2020/034221

| | | |
|---|---|---|
| US 2012/0128978 A1 | 24 May 2012 | WO 2010/125094 A2<br>entire text<br>DE 102009019370 A1 |
| JP 2017-518249 A | 06 July 2017 | US 2015/0315091 A1<br>claim 1, paragraphs [0074]-[0076]<br>WO 2015/168593 A1<br>CA 2947161 A1<br>KR 10-2017-0005423 A<br>CN 106458783 A |
| JP 2015-507529 A | 12 March 2015 | CA 2856785 A1<br>claim 1, examples 1-14<br>KR 10-2014-0107443 A<br>CN 104168994 A<br>WO 2013/092158 A2 |
| JP 2015-164722 A | 17 September 2015 | (Family: none) |

Form PCT/ISA/210 (patent family annex) (January 2015)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description

- JP 2015164722 A
- JP 2015507529 A

### Non-patent literature cited in the description

- Polyurethane Resin Handbook. The Nikkan Kogyo Shimbun, Ltd, 1987